# EUROPEAN PATENT SPECIFICATION

(11) **EP 3 488 860 B1**
(45) Date of publication and mention of the grant of the patent: **19.01.2022**
(21) Application number: 17382804.7
(22) Date of filing: 28.11.2017
(51) Int. Cl.: A61K 38/47, A61K 38/54, C12N 9/14, C12N 9/24

(54) **CHIMERIC PROTEIN WITH HIGH ANTIMICROBIAL ACTIVITY**
CHIMERES PROTEIN MIT HOHER ANTIMICROBIELLER AKTIVITÄT
PROTÉINE CHIMERIQUE À HAUTE ACTIVITÉ ANTIMICROBIENNE

(43) Date of publication of application: 29.05.2019
(73) Proprietor: Consejo Superior de Investigaciones Científicas (CSIC), 28006 Madrid (ES)
(72) Inventor: GUTIÉRREZ FERNÁNDEZ, Diana, 33300 Villaviciosa (ES); MARTÍNEZ FERNÁNDEZ, Beatriz, 33300 Villaviciosa (ES); GARCÍA SUÁREZ, Pilar, 33300 Villaviciosa (ES); RODRÍGUEZ GONZÁLEZ, Ana, 33300 Villaviciosa (ES); RODRÍGUEZ-RUBIO, Lorena, 08028 Barcelona (ES); LAVIGNE, Rob, 2180 Ekeren (BE)
(74) Representative: Pons Ariño, Angel

(56) References cited:
- WO-A2-2013/074959
- ELZBIETA JAGIELSKA ET AL: "LytM Fusion with SH3b-Like Domain Expands Its Activity to Physiological Conditions", MICROBIAL DRUG RESISTANCE., vol. 22, no. 6, 1 September 2016 (2016-09-01), pages 461-469, XP55440506, US ISSN: 1076-6294, DOI: 10.1089/mdr.2016.0053
- DWAYNE R ROACH ET AL: "Antimicrobial bacteriophage-derived proteins and therapeutic applications", BACTERIOPHAGE, vol. 5, no. 3, 23 June 2015 (2015-06-23), page e1062590, XP55420031, DOI: 10.1080/21597081.2015.1062590

### SEQUENCE LISTING

<110> Consejo Superior de Investigaciones Cientificas (CSIC)
<120> Chimeric protein with high antimicrobial activity
<130> EP1641.1323
<160> 8
<170> PatentIn version 3.5
<210> 1
   <211> 92
   <212> PRT
   <213> Staphylococcus phage phiIPLA-RODI
<400> 1
   Asp Ala Asp Gly Tyr Tyr His Ala Gln Cys Gln Asp Leu Ile Thr Asp
   1               5                   10                  15
   Tyr Val Leu Trp Leu Thr Asp Asn Lys Val Arg Thr Trp Gly Asn Ala
               20                  25                  30
   Lys Asp Gln Ile Lys Gln Ser Tyr Gly Thr Gly Phe Lys Ile His Glu
           35                  40                  45
   Asn Lys Pro Ser Thr Val Pro Lys Lys Gly Trp Ile Ala Val Phe Thr
       50                  55                  60
   Ser Gly Ser Tyr Gln Gln Trp Gly His Ile Gly Ile Val Tyr Asp Gly
   65                  70                  75                  80
   Gly Asn Thr Ser Thr Phe Thr Ile Leu Glu Gln Asn
                   85                  90
<210> 2
   <211> 165
   <212> PRT
   <213> Staphylococcus phage phiIPLA-RODI
<400> 2
   Met Ala Lys Thr Gln Ala Glu Ile Asn Lys Arg Leu Asp Ala Tyr Ala
   1               5                   10                  15
   Lys Gly Thr Val Asp Ser Pro Tyr Arg Ile Lys Lys Ala Thr Ser Tyr
               20                  25                  30
   Asp Pro Ser Phe Gly Val Met Glu Ala Gly Ala Ile Asp Ala Asp Gly
           35                  40                  45
   Tyr Tyr His Ala Gln Cys Gln Asp Leu Ile Thr Asp Tyr Val Leu Trp
   50 55 60
   Leu Thr Asp Asn Lys Val Arg Thr Trp Gly Asn Ala Lys Asp Gln Ile
   65 70 75 80
   Lys Gln Ser Tyr Gly Thr Gly Phe Lys Ile His Glu Asn Lys Pro Ser
   85 90 95
   Thr Val Pro Lys Lys Gly Trp Ile Ala Val Phe Thr Ser Gly Ser Tyr
   100 105 110
   Gln Gln Trp Gly His Ile Gly Ile Val Tyr Asp Gly Gly Asn Thr Ser
   115 120 125
   Thr Phe Thr Ile Leu Glu Gln Asn Trp Asn Gly Tyr Ala Asn Lys Lys
   130 135 140
   Pro Thr Lys Arg Val Asp Asn Tyr Tyr Gly Leu Thr His Phe Ile Glu
   145 150 155 160
   Ile Pro Val Lys Ala
   165
<210> 3
   <211> 66
   <212> PRT
   <213> Staphylococcus virus IPLA88
<400> 3
   Tyr Tyr Met Glu Glu Ser Ala Arg Phe Thr Asn Gly Asn Gln Pro Ile
   1 5 10 15
   Thr Val Arg Lys Val Gly Pro Phe Leu Ser Cys Pro Val Gly Tyr Gln
   20 25 30
   Phe Gln Pro Gly Gly Tyr Cys Asp Tyr Thr Glu Val Met Leu Gln Asp
   35 40 45
   Gly His Val Trp Val Gly Tyr Thr Trp Glu Gly Gln Arg Tyr Tyr Leu
   50 55 60
   Pro Ile
   65
<210> 4
   <211> 143
   <212> PRT
   <213> Staphylococcus virus IPLA88
<400> 4
   Leu Leu Pro Glu Asp Lys Arg Leu Gln Leu Lys Asp Tyr Phe Ile Lys
   1               5                   10                  15
   Gln Ile Arg Ala Tyr Met Asp Gly Lys Ile Pro Val Ala Thr Val Ser
               20                  25                  30
   Asn Asp Ser Ser Ala Ser Ser Asn Thr Val Lys Pro Val Ala Ser Ala
           35                  40                  45
   Trp Lys Arg Asn Lys Tyr Gly Thr Tyr Tyr Met Glu Glu Ser Ala Arg
       50                  55                  60
   Phe Thr Asn Gly Asn Gln Pro Ile Thr Val Arg Lys Val Gly Pro Phe
   65                  70                  75                  80
   Leu Ser Cys Pro Val Gly Tyr Gln Phe Gln Pro Gly Gly Tyr Cys Asp
                   85                  90                  95
   Tyr Thr Glu Val Met Leu Gln Asp Gly His Val Trp Val Gly Tyr Thr
               100                 105                 110
   Trp Glu Gly Gln Arg Tyr Tyr Leu Pro Ile Arg Thr Trp Asn Gly Ser
           115                 120                 125
   Ala Pro Pro Asn Gln Ile Leu Gly Asp Leu Trp Gly Glu Ile Ser
       130                 135                 140
<210> 5
   <211> 308
   <212> PRT
   <213> Artificial Sequence
<220>
   <223> Chimeric protein ClyRODI-H5
<400> 5
   Met Ala Lys Thr Gln Ala Glu Ile Asn Lys Arg Leu Asp Ala Tyr Ala
   1               5                   10                  15
   Lys Gly Thr Val Asp Ser Pro Tyr Arg Ile Lys Lys Ala Thr Ser Tyr
               20                  25                  30
   Asp Pro Ser Phe Gly Val Met Glu Ala Gly Ala Ile Asp Ala Asp Gly
           35                  40                  45
   Tyr Tyr His Ala Gln Cys Gln Asp Leu Ile Thr Asp Tyr Val Leu Trp
       50                  55                  60
   Leu Thr Asp Asn Lys Val Arg Thr Trp Gly Asn Ala Lys Asp Gln Ile
   65                  70                  75                  80
   Lys Gln Ser Tyr Gly Thr Gly Phe Lys Ile His Glu Asn Lys Pro Ser
                   85                  90                  95
   Thr Val Pro Lys Lys Gly Trp Ile Ala Val Phe Thr Ser Gly Ser Tyr
               100                 105                 110
   Gln Gln Trp Gly His Ile Gly Ile Val Tyr Asp Gly Gly Asn Thr Ser
           115                 120                 125
   Thr Phe Thr Ile Leu Glu Gln Asn Trp Asn Gly Tyr Ala Asn Lys Lys
       130                 135                 140
   Pro Thr Lys Arg Val Asp Asn Tyr Tyr Gly Leu Thr His Phe Ile Glu
   145                 150                 155                 160
   Ile Pro Val Lys Ala Leu Leu Pro Glu Asp Lys Arg Leu Gln Leu Lys
                   165                 170                 175
   Asp Tyr Phe Ile Lys Gln Ile Arg Ala Tyr Met Asp Gly Lys Ile Pro
               180                 185                 190
   Val Ala Thr Val Ser Asn Asp Ser Ser Ala Ser Ser Asn Thr Val Lys
           195                 200                 205
   Pro Val Ala Ser Ala Trp Lys Arg Asn Lys Tyr Gly Thr Tyr Tyr Met
       210                 215                 220
   Glu Glu Ser Ala Arg Phe Thr Asn Gly Asn Gln Pro Ile Thr Val Arg
   225                 230                 235                 240
   Lys Val Gly Pro Phe Leu Ser Cys Pro Val Gly Tyr Gln Phe Gln Pro
                   245                 250                 255
   Gly Gly Tyr Cys Asp Tyr Thr Glu Val Met Leu Gln Asp Gly His Val
               260                 265                 270
   Trp Val Gly Tyr Thr Trp Glu Gly Gln Arg Tyr Tyr Leu Pro Ile Arg
           275                 280                 285
   Thr Trp Asn Gly Ser Ala Pro Pro Asn Gln Ile Leu Gly Asp Leu Trp
       290                 295                 300
   Gly Glu Ile Ser
   305
<210> 6
   <211> 912
   <212> **DNA**
   <213> Artificial Sequence
<220>
   <223> polynucleotide encoding the chimeric protein ClyRODI-H5
<400> 6
   aaaacccagg cggagatcaa caagcgtctg gacgcgtacg cgaaaggtac cgtggatagc      60
   ccgtaccgta ttaagaaagc gaccagctat gatccgagct tcggtgttat ggaagcgggc     120
   gcgatcgacg cggatggtta ctatcacgcg cagtgccaag acctgattac cgattacgtg     180
   ctgtggctga ccgacaacaa ggttcgtacc tggggcaacg cgaaagatca gatcaagcaa     240
   agctatggta ccggcttcaa aattcacgag aacaagccga gcaccgtgcc gaagaaaggt     300
   tggatcgcgg tttttaccag cggcagctac cagcaatggg gccacatcgg tattgtgtat     360
   gatggtggca acaccagcac cttcaccatt ctggaacaga actggaacgg ttacgcgaac     420
   aagaaaccga ccaaacgtgt tgacaactac tatggtctga cccactttat cgagattccg     480
   gtgaaggcgc tgctgccgga agataaacgc ctgcagctga aggactattt tattaaacaa     540
   atccgtgcgt acatggatgg caagattccg gtcgcgaccg tgtcaaacga cagttccgcc     600
   tcatcgaata cggttaaacc ggtcgcctcg gcatggaaac gcaataagta tggtacctat     660
   tacatggaag aaagcgcccg ttttaccaac ggcaatcagc cgatcacggt gcgcaaagtt     720
   ggtccgtttc tgagctgccc ggtcggctat cagttccaac cgggtggcta ttgtgattac     780
   accgaagtga tgctgcagga cggccatgtt tgggtcggtt acacgtggga aggccagcgt     840
   tattacctgc cgattcgcac ctggaatggc tccgctccgc cgaatcaaat cctgggtgac     900
   ctgtggggtg aa                                                         912
<210> 7
   <211> 496
   <212> PRT
   <213> Staphylococcus phage philPLA-RODI
<400> 7
   Met Ala Lys Thr Gln Ala Glu Ile Asn Lys Arg Leu Asp Ala Tyr Ala
   1               5                   10                  15
   Lys Gly Thr Val Asp Ser Pro Tyr Arg Ile Lys Lys Ala Thr Ser Tyr
               20                  25                  30
   Asp Pro Ser Phe Gly Val Met Glu Ala Gly Ala Ile Asp Ala Asp Gly
           35                  40                  45
   Tyr Tyr His Ala Gln Cys Gln Asp Leu Ile Thr Asp Tyr Val Leu Trp
       50                  55                  60
   Leu Thr Asp Asn Lys Val Arg Thr Trp Gly Asn Ala Lys Asp Gln Ile
   65                  70                  75                  80
   Lys Gln Ser Tyr Gly Thr Gly Phe Lys Ile His Glu Asn Lys Pro Ser
                   85                  90                  95
   Thr Val Pro Lys Lys Gly Trp Ile Ala Val Phe Thr Ser Gly Ser Tyr
               100                 105                 110
   Gln Gln Trp Gly His Ile Gly Ile Val Tyr Asp Gly Gly Asn Thr Ser
           115                 120                 125
   Thr Phe Thr Ile Leu Glu Gln Asn Trp Asn Gly Tyr Ala Asn Lys Lys
       130                 135                 140
   Pro Thr Lys Arg Val Asp Asn Tyr Tyr Gly Leu Thr His Phe Ile Glu
   145                 150                 155                 160
   Ile Pro Val Lys Ala Gly Thr Thr Val Lys Lys Glu Thr Ala Lys Lys
                   165                 170                 175
   Ser Ala Ser Lys Thr Pro Ala Pro Lys Lys Lys Ala Thr Leu Lys Val
               180                 185                 190
   Ser Lys Asn His Ile Asn Tyr Thr Met Asp Lys Arg Gly Lys Lys Pro
           195                 200                 205
   Glu Gly Met Val Ile His Asn Asp Ala Gly Arg Ser Ser Gly Gln Gln
       210                 215                 220
   Tyr Glu Asn Ser Leu Ala Asn Ala Gly Tyr Ala Arg Tyr Ala Asn Gly
   225                 230                 235                 240
   Ile Ala His Tyr Tyr Gly Ser Glu Gly Tyr Val Trp Glu Ala Ile Asp
                   245                 250                 255
   Ala Lys Asn Gln Ile Ala Trp His Thr Gly Asp Gly Thr Gly Ala Asn
               260                 265                 270
   Ser Gly Asn Phe Arg Phe Ala Gly Ile Glu Val Cys Gln Ser Met Ser
           275                 280                 285
   Ala Ser Asp Ala Gln Phe Leu Lys Asn Glu Gln Ala Val Phe Gln Phe
       290                 295                 300
   Thr Ala Glu Lys Phe Lys Glu Trp Gly Leu Thr Pro Asn Arg Lys Thr
   305 310 315 320
   Val Arg Leu His Met Glu Phe Val Pro Thr Ala Cys Pro His Arg Ser
   325 330 335
   Met Val Leu His Thr Gly Phe Asn Pro Val Thr Gln Gly Arg Pro Ser
   340 345 350
   Gln Ala Ile Met Asn Lys Leu Lys Asp Tyr Phe Ile Lys Gln Ile Lys
   355 360 365
   Asn Tyr Met Asp Lys Gly Thr Ser Ser Ser Thr Ile Val Lys Asp Gly
   370 375 380
   Lys Thr Ser Ser Ala Ser Thr Pro Ala Thr Arg Pro Val Thr Gly Ser
   385 390 395 400
   Trp Lys Lys Asn Gln Phe Gly Thr Trp Tyr Lys Pro Glu Ser Ala Thr
   405 410 415
   Phe Val Asn Gly Asn Gln Pro Ile Ile Thr Arg Ile Gly Ser Pro Phe
   420 425 430
   Leu Asn Ala Pro Ile Gly Gly Asn Leu Pro Ala Gly Ala Thr Ile Val
   435 440 445
   Tyr Asp Glu Val Cys Ile Gln Ala Gly His Ile Trp Ile Gly Tyr Asn
   450 455 460
   Ala Tyr Asn Gly Asn Arg Val Tyr Cys Pro Val Arg Thr Cys Gln Gly
   465 470 475 480
   Val Pro Pro Asn His Ile Pro Gly Val Ala Trp Gly Val Phe Lys Gly
   485 490 495
<210> 8
   <211> 486
   <212> **PRT**
   <213> Staphylococcus virus IPLA88
<400> 8
   Met Glu Val Ala Thr Met Gln Ala Lys Leu Thr Lys Lys Glu Phe Ile
   1 5 10 15
   Glu Trp Leu Lys Thr Ser Glu Gly Lys Gln Tyr Asn Ala Asp Gly Trp
   20 25 30
   Tyr Gly Phe Gln Cys Phe Asp Tyr Ala Asn Ala Gly Trp Lys Ala Leu
           35                  40                  45
   Phe Gly Leu Leu Leu Lys Gly Val Gly Ala Lys Asp Ile Pro Phe Ala
       50                  55                  60
   Asn Asn Phe Asp Gly Leu Ala Thr Val Tyr Gln Asn Thr Pro Asp Phe
   65                  70                  75                  80
   Leu Ala Gln Pro Gly Asp Met Val Val Phe Gly Ser Asn Tyr Gly Ala
                   85                  90                  95
   Gly Tyr Gly His Val Ala Trp Val Ile Glu Ala Thr Leu Asp Tyr Ile
               100                 105                 110
   Ile Val Tyr Glu Gln Asn Trp Leu Gly Gly Gly Trp Thr Asp Gly Val
           115                 120                 125
   Gln Gln Pro Gly Ser Gly Trp Glu Lys Val Thr Arg Arg Gln His Ala
       130                 135                 140
   Tyr Asp Phe Pro Met Trp Phe Ile Arg Pro Asn Phe Lys Ser Glu Thr
   145                 150                 155                 160
   Ala Pro Arg Ser Val Gln Ser Pro Thr Gln Ala Ser Lys Lys Glu Thr
                   165                 170                 175
   Ala Lys Pro Gln Pro Lys Ala Val Glu Leu Lys Ile Ile Lys Asp Val
               180                 185                 190
   Val Lys Gly Tyr Asp Leu Pro Lys Arg Gly Ser Asn Pro Asn Phe Ile
           195                 200                 205
   Val Ile His Asn Asp Ala Gly Ser Lys Gly Ala Thr Ala Glu Ala Tyr
       210                 215                 220
   Arg Asn Gly Leu Val Asn Ala Pro Leu Ser Arg Leu Glu Ala Gly Ile
   225                 230                 235                 240
   Ala His Ser Tyr Val Ser Gly Asn Thr Val Trp Gln Ala Leu Asp Glu
                   245                 250                 255
   Ser Gln Val Gly Trp His Thr Ala Asn Gln Ile Gly Asn Lys Tyr Gly
               260                 265                 270
   Tyr Gly Ile Glu Val Cys Gln Ser Met Gly Ala Asp Asn Ala Thr Phe
           275                 280                 285
   Leu Lys Asn Glu Gln Ala Thr Phe Gln Glu Cys Ala Arg Leu Leu Lys
       290                 295                 300
   Lys Trp Gly Leu Pro Ala Asn Arg Asn Thr Ile Arg Leu His Asn Glu
   305                 310                 315                 320
   Phe Thr Ser Thr Ser Cys Pro His Arg Ser Ser Val Leu His Thr Gly
                   325                 330                 335
   Phe Asp Pro Val Thr Arg Gly Leu Leu Pro Glu Asp Lys Arg Leu Gln
               340                 345                 350
   Leu Lys Asp Tyr Phe Ile Lys Gln Ile Arg Ala Tyr Met Asp Gly Lys
           355                 360                 365
   Ile Pro Val Ala Thr Val Ser Asn Asp Ser Ser Ala Ser Ser Asn Thr
       370                 375                 380
   Val Lys Pro Val Ala Ser Ala Trp Lys Arg Asn Lys Tyr Gly Thr Tyr
   385                 390                 395                 400
   Tyr Met Glu Glu Ser Ala Arg Phe Thr Asn Gly Asn Gln Pro Ile Thr
                   405                 410                 415
   Val Arg Lys Val Gly Pro Phe Leu Ser Cys Pro Val Gly Tyr Gln Phe
               420                 425                 430
   Gln Pro Gly Gly Tyr Cys Asp Tyr Thr Glu Val Met Leu Gln Asp Gly
           435                 440                 445
   His Val Trp Val Gly Tyr Thr Trp Glu Gly Gln Arg Tyr Tyr Leu Pro
       450                 455                 460
   Ile Arg Thr Trp Asn Gly Ser Ala Pro Pro Asn Gln Ile Leu Gly Asp
   465                 470                 475                 480
   Leu Trp Gly Glu Ile Ser
                   485

## Description

The invention relates to a chimeric protein with an improved lytic activity against *Staphylococcus* sp. and a higher stability than the parental endolysins of the state of the art. Thus, the invention relates to this chimeric protein and its use as an antimicrobial, being useful for treating and/or preventing an infection of *Staphylococcus* sp., for removing *Staphylococcus* sp. biofilms, for controlling the *Staphylococcus* sp. contamination in food industry and for decontaminating inanimate surfaces suspected of containing *Staphylococcus* sp. Thus, the present invention belongs to the field of the treatment of bacterial contamination or infections.

### BACKGROUND ART

The rapid rise and dissemination of multi-drug resistant (MDR) pathogens represents a major global long-term threat to human health, sustainable food production and development. According to the World Health Organization (WHO), the Food and Agriculture Organization of the United Nations (FAO) and the World Organization for Animal Health (OIE), MDR pathogens cause more than 700,000 deaths per year in the world, affecting developing and developed countries, rural and urban areas, hospitals, farms and communities. The emergence of MDR pathogens is an urgent global risk that remains unsolved.

*Staphylococcus aureus* is considered as Priority 2 (high) in the global priority pathogens list (global PPL) of antibiotic-resistant bacteria elaborated by WHO. This bacterium is one of the major bacterial agents causing foodborne diseases in humans due to their ability to produce enterotoxins and it is also a serious threat for human health. The ability of S. *aureus* to adhere to and proliferate not only on abiotic surfaces but also on human tissues makes this bacterium difficult to eradicate.

Over the last years, the study of bacteriophage-encoded lytic proteins (endolysins and virion-associated peptidoglycan hydrolases) has attracted great interest for the treatment of infectious diseases. These enzymes kill the bacteria through peptidoglycan degradation followed by osmotic lysis. Indeed, there is evidence that many phage lytic proteins (enzybiotics) are effective in animal models of infection. The endolysin LysH5 (481 amino acids) encoded by phage vB_SauS-philPLA88 (phiIPLA88) has three putative domains, an N-terminal cysteine, histidine-dependent amidohydrolases/peptidase (CHAP) domain, and amidase-2 domain, and a C-terminal SH3b cell wall-binding (CWB) domain. LysH5 is able to inhibit *S*. *aureus* growth in milk and shows a synergistic antimicrobial effect with bacteriocin nisin. The phage philPLA88 virion-associated peptidoglycan hydrolase (HydH5, 634 amino acids) has an N-terminal CHAP lytic domain and a C-terminal LYZ2 (lysozyme family 2) lytic domain. Rodriguez-Rubio, L. et al. 2013 (PLOS ONE, 8(5): e64671) disclose two fusion proteins (HydH5SH3b and HydH5Lyso) obtained by combination between lysostaphin and HydH5 domains. These proteins exhibited high lytic activity against bovine and human *S*. *aureus.*

The international application WO2013/188203 A1 discloses the staphylococcal phage 2638A endolysin having lytic activity against *S*. *aureus* when exposed externally, making it a new antimicrobial candidate.

The international application WO2013/074959 A2 discloses three different fusion proteins: HydH5 peptidoglycan hydrolase-lysostaphin, HydH5 peptidoglycan hydrolase-SH3b and HydH5CHAP-SH3b. Each recombinant fusion polypeptide showed higher staphylolytic activity than the parental enzyme or its deletion construct. Both parental and fusion proteins lysed *S*. *aureus* cells in zymograms, plate-lysis and turbidity-reduction assays.

The patent US8481289 B2 discloses an antimicrobial endolysin-Lysostaphin triple fusion antimicrobial protein comprising (1) an endolysin CHAP endopeptidase, (2) an endolysin amidase domain, and (3) a Lysostaphin glycyl-glycine endopeptidase domain. The protein has specificity and exolytic activity for the peptidoglycan cell wall of untreated, live S. *aureus* from different growth phases.

However, there is still a necessity of providing new antimicrobial alternatives with improved killing activity against *Staphylococcus* sp, preferably, *S*. *aureus,* more preferably, *S*. *aureus* MDR.

### DESCRIPTION OF THE INVENTION

*Staphylococcus aureus* is one of the major bacterial agents causing foodborne diseases in humans due to its ability to produce enterotoxins and to adhere to and proliferate not only on abiotic surfaces but also on human tissues. Now, the authors of the present invention have discovered that a specific combination of the catalytic (CHAP) and cell wall binding domain (SH3b) derived from two endolysins gives raise, surprisingly, to a chimeric protein with an improved lytic activity against *Staphylococcus* sp. and a higher stability than the parental endolysins. The invention discloses the fusion of the CHAP domain of the endolysin LysRODI from phage PhiIPLA-RODI to the SH3b domain of the endolysin LysH5 from the phage phiIPLA88.

In order to do this, the inventors identified and fused the CHAP domain of the parental endolysin LysRODI and the SH3b domain of the parental endolysin LysH5, resulting in a chimeric protein which was subjected to several tests for analysing its antimicrobial activity and its capacity for preventing biofilm formation and for removing the pre-formed biofilms. As can be seen in the illustrative example of the invention (Example 1), the above-mentioned chimeric protein showed an increased activity compared to the parental endolysins (Figure 1), a *S*. *aureus* bacteria killing rate similar to the parental endolysins (Figure 2) and increased anti-biofilm properties (Figures 3 and 4). Furthermore, the chimeric protein herein disclosed does not cause *Staphylococcus* sp. resistance, maintains its activity from pH 5 to pH 11 allowing the topic use thereof, and its purification is easier than other chimeric proteins. These features turn the chimeric protein of the present invention into a new antimicrobial agent useful in the treatment of *Staphylococcus* sp. diseases, the biocontrol of foods and the disinfection of surfaces.

### Chimeric protein of the invention

In one aspect, the present invention relates to a chimeric protein, hereinafter "chimeric protein of the invention", comprising:
(a) a first amino acid sequence comprising the CHAP catalytic domain of the endolysin LysRODI from phage PhiIPLA-RODI, and
(b) a second amino acid sequence comprising the SH3b cell wall binding domain of the endolysin LysH5 from phage phiIPLA88,
wherein the first amino acid sequence (a) is bound to the second amino acid sequence (b) and wherein the (i) stability and (ii) lytic activity of the chimeric protein against *Staphylococcus sp.* are improved when compared to those of the parental endolysins.

The term "chimeric protein", as used herein, refers to any protein comprised of a first amino acid sequence derived from a first source, bound, covalently or non-covalently, to a second amino acid sequence derived from a second source, wherein the first and second source are not the same. A first source and a second source that are not the same can include two different biological entities. A chimeric protein can include, for example, a protein derived from at least 2 different biological sources. The source may also be a synthetic source. A synthetic source can include a protein or nucleic acid sequence produced chemically and not by a biological system (e.g. solid phase synthesis of amino acid sequences). Thus, the term "chimeric protein" means a protein or polypeptide comprising two or more heterologous domains which are not found in the same relationship to one another in nature.

The chimeric protein of the invention comprises a first amino acid sequence (sequence (a)) comprising the CHAP catalytic domain of the endolysin LysRODI from phage philPLA-RODI.

The endolysin LysRODI (also called LysRODI) is encoded by the *orf*57 from the phage vB-SauM_phiIPLA-RODI (also called *Staphylococcus* phage phiIPLA-RODI). The endolysin LysRODI shows the amino acid sequence SEQ ID NO: 7 (Genbank accession number YP_009195893.1).

### SEQ ID NO: 7

The endolysin LysRODI comprises two catalytic domains: the CHAP domain [SEQ ID NO: 1: amino acids 45 to 136 of SEQ ID NO: 7 (underlined)] and the Amidase_2 domain (amino acids 204 to 335 of SEQ ID NO: 7), and one cell wall binding domain SH3b (amino acids 409 to 475 of SEQ ID NO: 7).

Thus, in a particular embodiment of the chimeric protein of the invention, the amino acid sequence of the catalytic domain CHAP comprises an amino acid sequence with a sequence identity of at least 80, 85, 90, 95, 96, 97, 98, 99 or 100% to SEQ ID NO: 1.

### SEQ ID NO: 1

In the present invention, the terms "identical", "sequence identity", "identity" and "similarity" are considered equivalents and can be used interchangeably. The term "sequence identity" is understood to mean the degree of similarity between two nucleotide or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programmes, for example, BLASTN, BLASTX, and TBLASTX, BLASTP and TBLASTN, are in the public domain at The National Center for Biotechonology Information (NCBI) website. Proteins with the above-mentioned sequence identity to SEQ ID NO: 1 are considered variants of SEQ ID NO: 1 and are also contemplated within the present invention.

In another particular embodiment, the first amino acid sequence of the chimeric protein of the invention comprises the sequence SEQ ID NO: 2.

### SEQ ID NO: 2

The chimeric protein of the invention also comprises a second amino acid sequence (sequence (b) comprising the SH3b cell wall binding domain of the endolysin LysH5 from phage phiIPLA88.

The endolysin LysH5 (also called LysH5) is encoded by the *orf61* from the phage vB-SauS_phiIPLA88 (also called *Staphylococcus* virus IPLA88). The endolysin LysH5 shows the amino acid sequence SEQ ID NO: 8 (Genbank accession number YP_002332536.1).

### SEQ ID NO: 8

The endolysin LysH5 comprises two catalytic domains: the CHAP domain (amino acids 28 to 118 of SEQ ID NO: 8) and the Amidase_2 domain (amino acids 203 to 327 of SEQ ID NO: 8), and one SH3b cell wall binding domain (SEQ ID NO: 3: amino acids 400 to 465 of SEQ ID NO: 8).

Thus, in a particular embodiment of the chimeric protein of the invention, the amino acid sequence of the SH3b cell wall binding domain comprises an amino acid sequence with a sequence identity of at least 80, 85, 90, 95, 96, 97, 98, 99 or 100% to SEQ ID NO: 3.

### SEQ ID NO: 3

The term "sequence identity" has been previously disclosed in the present description.

In another particular embodiment, the second amino acid sequence of the chimeric protein of the invention comprises the amino acid sequence SEQ ID NO: 4.

### SEQ ID NO: 4

In another particular embodiment, the chimeric protein (ClyRODI-H5) comprises the amino acid sequence SEQ ID NO: 5.

As is well known to those skilled in the art, altering the primary structure of a protein by a conservative substitution may not significantly alter the activity of that peptide because the side-chain of the amino acid which is inserted into the sequence may be able to form similar bonds and contacts as the side chain of the amino acid which has been substituted out. Non-conservative substitutions are possible provided that these do not interrupt or interfere with the function of the chimeric protein of the invention.

As used herein, the terms "variant", "analog" and "derivative" are equivalents and refer to a protein comprising at least one altered amino acid residue by an amino acid substitution, addition, deletion or chemical modification, as compared with the native protein, but always showing the catalytic activity of SEQ ID NO: 5 (the chimeric protein of the invention). Assays for checking the function of SEQ ID NO: 5 variants can be found in the Example of the present description, such as turbidity reduction assay and time killing assay. Peptide derivatives particularly include amino acid substitutions and/or additions with naturally occurring amino acid residues, and chemical modifications such as, for example, enzymatic modifications, typically present in nature. Proteins variants particularly include amino acid substitutions and/or additions with non-natural amino acid residues, and chemical modifications which do not occur in nature. Broadly speaking, fewer non-conservative substitutions will be possible without altering the biological activity of the chimeric protein of the invention. Thus, functional mutants, variants or derivatives of the above-mentioned chimeric protein are also covered by the present invention. A given protein is considered a variant of the chimeric protein of the invention if said protein shows an sequence identity of, at least, 80, 85, 90, 95, 96, 97, 98 or 99% with SEQ ID NO: 5. The term "sequence identity" has been previously defied. Such mutants, variants or derivatives are considered to be "functional" if they retain the same or similar properties and/or activity to the chimeric protein of the invention as described herein. A variant of the chimeric protein of the invention is considered functional where the level of activity is at least 80%, yet more preferably 90%, 95%, 96%, 97%, 98%, 99% or 100% that of the chimeric protein. The skilled person is routinely able to determine such properties and activities and examples of such methods are provided in the current specification and mentioned above.

The first and second amino acid sequences of the chimeric protein of the invention can be covalently o non-covalently bound to each other. The terms "bound to", "couple to" and "linked to" are considered equivalents and can be used interchangeably throughout the present description.

The expression "covalently bound to each other" means that the two amino acid sequences are bound by a bond that involves the sharing of electron pairs between atoms, such as a peptide bond. On the contrary, the expression "non-covalently bound to each other" means that the two amino acid sequences are bound by a bond in that it does not involve the sharing of electrons, but rather involves more dispersed variations of electromagnetic interactions between molecules or within a molecule.

Additionally, the first and second amino acids sequences of the chimeric protein of the invention may be bound by a linker. A "linker" or "peptide linker" refers to a peptide sequence linked to either the N-terminus or the C-terminus of a polypeptide sequence. Any "linker" group is optional. When present, its chemical structure is not critical, since it serves primarily as a spacer. The linker is preferably made up of amino acids linked together by peptide bonds. Thus, in preferred embodiments, the linker is made up of from 1 to 20 amino acids linked by peptide bonds, wherein the amino acids are selected from the 20 naturally occurring amino acids. Some of these amino acids may be glycosylated, as is well understood by those in the art. In a more preferred embodiment, the 1 to 20 amino acids are selected from glycine, alanine, proline, asparagine, glutamine, and lysine. Even more preferably, a linker is made up of a majority of amino acids that are sterically unhindered, such as glycine and alanine. In order to ensure proper folding of the protein, these linkers should not contain truncated α-helices or β-sheets.

In a particular embodiment, the chimeric protein is preferably a soluble protein.

The chimeric protein described herein may be conjugated to a stabilizing molecule. Non-limiting examples of stabilizing molecules include: a polymer (e.g., a polyethylene glycol) or a protein (e.g., serum albumin, such as feline serum albumin). Likewise, the chimeric protein may also be conjugated to a label (e.g., a fluorophore, radioisotope, or luminescent molecule) or a therapeutic agent (e.g., a cytotoxic agent or a radioisotope). Non-limiting examples of agents that can be conjugated to or combined with the chimeric protein of the invention include, without limiting to: linezolid, erythromycin, mupirocin, ertapenem, doripenem, imipenem, cilastatin, meropenem, cefadroxil, cefazolin, cefalotin, cephalothin, cephalexin, ceflacor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, ceftaroline fosamil, ceftobiprole, teicoplanin, vancomycin, televancin, clindamycin, lincomycin, daptomycin, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, penicillin G, temocillin, ticarcillin, bacitracin, colistin, polymyxin B, ciprofloxacin, enoxacin, gatifloxacin, gemifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sufamethizole, sulfamethoxazole, sulfanilamide, sulfasalazine, sulfisoxazole, trimethoprim-sulfamethoxazole, sulfonamidochrysoidine, demeclocycline, doxycycline, minocycline, oxytetracycline, and tetracycline.

Additionally, the chimeric protein of the invention may be engineered to increase its lytic activity, its stability at high temperatures and its host range. With this purpose, the chimeric protein can be modified by a technique called "artilysation". This technique comprises fusing to either the N- or C- terminus of the chimeric protein of the invention an additional cationic or polycationic peptide, an amphipathic peptide, a sushi peptide, a defensin, a hydrophobic peptide or an antimicrobial peptide.

The term "peptide" as used herein refers to short polypeptides consisting from about 2 to about 100 amino acid residues, more preferably from about 4 to about 50 amino acid residues, more preferably from about 5 to 30 amino acid residues, wherein the amino group of one amino acid residue is linked to the carboxyl group of another amino acid residue by a peptide bond. A peptide can be a naturally occurring peptide or a synthetically designed and produced peptide. The peptide can be, for example, derived or removed from a native protein by enzymatic or chemical cleavage, or can be prepared using conventional peptide synthesis techniques (e.g., solid phase synthesis) or molecular biology techniques (see Sambrook, J. et al., Molecular Cloning: A Laboratory Manual, Cold Spring Harbor Press, Cold Spring Harbor, N.Y. (1989)). Preferred synthetically produced peptides are e.g. cationic, polycationic peptides, amphipathic peptides and hydrophobic peptides. Preferred naturally occurring peptides are e.g. amphipathic peptides, sushi peptides, defensins and antimicrobial peptides. The peptides have no or only low cell wall degrading activity but may have cell wall disrupting activity. A peptide in the meaning of the present invention does not refer to His-tags, Strep-tags, thioredoxin or maltose binding proteins (MBP) or the like, which are used to purify or locate proteins.

As used herein, the term "cationic peptide" refers to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides. Examples of polycationic peptides include, without limiting to, a LPS-destabilizing peptide (Briers, Y., et al. 2014. mBio, 5(4): e01379-14; Germans et al. 2016. Biochem.Soc.Trans. 44:123-128) and sheep myeloid 29-amino acid (SMAP-29) peptide (Skerlavaj B, et al. 1999. FEBS Lett. 46:58-62).

The term "polycationic peptide" as used herein refers to a synthetically designed and produced peptide composed of mostly positively charged amino acid residues, in particular lysine, arginine and/or histidine residues, more preferably lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues.

The term, "antimicrobial peptide" (AMP) as used herein refers to any naturally occurring peptide that has microbicidal and/or microbiostatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, antiparasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. The antimicrobial peptide may be a member of the RNAse A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in *Xenopus laevis, Rana frogs,* more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human. The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphipathic peptide" as used herein refers to synthetic peptides having both hydrophilic and hydrophobic functional groups. Preferably, the term "amphipathic peptide" as used herein refers to a peptide having a defined arrangement of hydrophilic and hydrophobic groups e.g. amphipathic peptides may be e.g. alpha helical, having predominantly non polar side chains along one side of the helix and polar residues along the remainder of its surface.

The term "hydrophobic group" as used herein refers to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a non-aqueous environment. Examples of amino acid residues with hydrophobic side chains are valine, isoleucine, leucine, methionine, phenylalanine, tryptophan, cysteine, alanine, tyrosine, histidine, threonine, serine, proline and glycine residues.

In another aspect, the invention relates to an isolated polynucleotide, hereinafter "polynucleotide of the invention", comprising a nucleotide sequence encoding the chimeric protein of the invention. Thus, the polynucleotide of the invention is the coding sequence of the chimeric protein of the invention. The term "coding sequence" means a polynucleotide which directly specifies the amino acid sequence of a protein. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide.

In order to express the polynucleotide encoding the chimeric protein of the invention, the nucleotide sequence has to be introduced in an expression system such as a host cell. Thus, the nucleotide sequence may be optimized for a proper expression in the chosen host cell. In the present invention, the nucleotide sequence encoding the chimeric protein of the invention is optimized for its expression in *Escherichia coli.* Thus, in a particular embodiment, the polynucleotide of the invention comprises the sequence SEQ ID NO: 6.

### SEQ ID NO: 6

The polynucleotide of the invention can be inserted into a nucleic acid construct. The term "nucleic acid construct" means a nucleic acid molecule, either single- or double-stranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains control sequences required for expression of the polynucleotide of the invention.

The term "control sequences" means all components necessary for the expression of a polynucleotide encoding a polypeptide or protein, and which are placed at an appropriate position relative to the coding sequence of a polynucleotide such that the control sequence directs the expression of the polynucleotide. The term "expression" includes any step involved in the production of the chimeric protein of the invention including, but not limited to, transcription, post-transcriptional modification, translation, post-translational modification, and secretion.

Each control sequence may be native or foreign to the polynucleotide of the invention or native or foreign to each other. Such control sequences include, but are not limited to, a leader, polyadenylation sequence, propeptide sequence, promoter, signal peptide sequence, and transcription terminator. At a minimum, the control sequences include a promoter and transcriptional and translational stop signals. The control sequences may be provided with linkers for the purpose of introducing specific restriction sites facilitating ligation of the control sequences with the coding region of the polynucleotide of the invention.

The control sequence may be an appropriate promoter sequence, i.e. a nucleotide sequence that is recognized by a host cell for expression of the polynucleotide of the invention. A "promoter," as used herein, refers to a nucleotide sequence that directs the transcription of a gene or coding sequence to which it is operably linked.

"Operably linked" refers to an arrangement of two or more components, wherein the components so described are in a relationship permitting them to function in a coordinated manner. By way of illustration, a transcriptional regulatory sequence or a promoter is operably linked to a coding sequence if the transcriptional regulatory sequence or promoter facilitates some aspect of the transcription of the coding sequence. Aspects of the transcription process include, but are not limited to, initiation, elongation, attenuation and termination. An operably linked transcriptional regulatory sequence is generally joined in cis with the coding sequence, but it is not necessarily directly adjacent to it. The promoter sequence contains transcriptional control sequences that mediate the expression of the polypeptide. The promoter may be any nucleotide sequence that shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the polynucleotide of the present invention in a bacterial host cell include, without limiting to, the promoters obtained from the T7 phage, the *E. coli* lac operon, *Streptomyces coelicolor* agarase gene (dagA), *Bacillus subtilis* levansucrase gene (sacB), *Bacillus licheniformis* alpha-amylase gene (amyL), *Bacillus stearothermophilus* maltogenic amylase gene (amyM), *Bacillus amyloliquefaciens* alpha-amylase gene (amyQ), *Bacillus licheniformis* penicillinase gene (penP), *Bacillus subtilis* xylA and xylB genes, and prokaryotic beta-lactamase gene, as well as the tac promoter. In a particular embodiment, the promoter is the promoter 7 of the *E*. *coli pfl* operon.

Examples of suitable promoters for directing the transcription of the nucleic acid constructs in a filamentous fungal host cell include, without limiting to, promoters obtained from the genes for *Aspergillus nidulans* acetamidase, *Aspergillus niger* neutral alpha-amylase, *A*. *niger* acid stable alpha-amylase, *A*. *niger* or *Aspergillus awamori* glucoamylase (glaA), *A*. *oryzae* TAKA amylase, *A.oryzae* alkaline protease, *A*. *oryzae* triose phosphate isomerase, *Fusarium oxysporum* trypsin-like protease, *Fusarium venenatum* amyloglucosidase, *Fusarium venenatum* Daria, *Fusarium venenatum* Quinn, *Rhizomucor miehei* lipase, *R. miehei* aspartic proteinase, *Trichoderma reesei* beta-glucosidase, *T. reesei* cellobiohydrolase I, *T. reesei* cellobiohydrolase II, *T. reesei* endoglucanase I, *T. reesei* endoglucanase II, *T. reesei* endoglucanase III, *T. reesei* endoglucanase IV, *T. reesei* endoglucanase V, *T. reesei* xylanase I, *T. reesei xylanase* II, *T. reesei* beta*-* xylosidase, as well as the NA2-tpi promoter (a modified promoter from a gene encoding a neutral alpha-amylase in Aspergilli in which the untranslated leader has been replaced by an untranslated leader from a gene encoding triose phosphate isomerase in Aspergilli; non-limiting examples include modified promoters from the gene encoding neutral alpha-amylase in *A. niger* in which the untranslated leader has been replaced by an untranslated leader from the gene encoding triose phosphate isomerase in *A. nidulans* or *A. oryzae);* and mutant, truncated, and hybrid promoters thereof.

In a yeast host, useful promoters are obtained from, without limiting to, the genes for S. *cerevisiae* enolase (ENO-1), *S*. *cerevisiae* galactokinase (GAL1), *Saccharomyces cerevisiae* alcohol dehydrogenase/glyceraldehyde-3-phosphate dehydrogenase (ADH1, ADH2/GAP), *S*. *cerevisiae* triose phosphate isomerase (TPI), *S*. *cerevisiae* metallothionein (CUP1), and *S*. *cerevisiae* 3-phosphoglycerate kinase.

Examples of suitable promoters for directing the transcription of the polynucleotide of the invention in a mammal host cell include, without limiting to, SV40 promoter (Mulligan, et al., Nature 277: 108-14 (1979)), MMLV-LTR promoter, EF1α promoter (Mizushima, et al., Nucleic Acids Res 18: 5322 (1990)), or CMV promoter.

Examples of suitable promoters for directing the transcription of the polynucleotide of the invention in a plant host cell include, without limiting to, the cauliflower mosaic virus (CaMV) 35S promoter or its enhanced version, the hybrid (ocs) 3mas promoter, the ubiquitin promoter from Maize, *A. thaliana* promoter and the rice α-amylase RAmy3D promoter which is induced by sugar deprivation.

The control sequence may also be a suitable transcription terminator sequence, a sequence recognized by a host cell to terminate transcription. The terminator sequence is operably linked to the 3' terminus of the nucleotide sequence encoding the chimeric protein of the invention. Any terminator that is functional in the host cell of choice may be used in the present invention.

Terminators for filamentous fungal host cells, included, without limited to, terminators obtained from the genes for *Aspergillus nidulans* anthranilate synthase, *Aspergillus niger* glucoamylase, *A. niger* alpha-glucosidase, *Aspergillus oryzae* TAKA amylase, and *Fusarium oxysporum* trypsin-like protease.

Terminators for yeast host cells included, without limiting to, terminators obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase.

The control sequence may also be a suitable leader sequence, a non-translated region of an mRNA that is important for translation by the host cell. The leader sequence is operably linked to the 5' terminus of the nucleotide sequence encoding the chimeric protein. Any leader sequence that is functional in the host cell of choice may be used in the present invention.

The control sequence may also be a polyadenylation sequence, a sequence operably linked to the 3' terminus of the nucleotide sequence and, when transcribed, is recognized by the host cell as a signal to add polyadenosine residues to transcribed mRNA. Any polyadenylation sequence that is functional in the host cell of choice may be used in the present invention.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. The foreign signal peptide coding sequence may be required where the coding sequence does not naturally contain a signal peptide coding sequence. Any signal peptide coding sequence that directs the expressed chimeric protein into the secretory pathway of a host cell of choice may be used.

It may also be desirable to add regulatory sequences that allow the regulation of the expression of the polynucleotide relative to the growth of the host cell. Examples of regulatory systems are those that cause the expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the chimeric protein would be operably linked with the regulatory sequence.

The various nucleic acids and control sequences described herein may be joined together to construct a recombinant expression vector that may include one or more (several) convenient restriction sites to allow for insertion or substitution of the polynucleotide of the invention encoding the chimeric protein of the invention. Accordingly, the polynucleotide of the invention may comprise enzymatic restriction sites in the ends of the nucleotide sequence which allow its insertion into a suitable vector. In a particular embodiment, the polynucleotide of the invention comprises a restriction site in the 5' end corresponding to the *Nde*I restriction enzyme and/or a restriction site in the 3' end of the nucleotide sequence corresponding to the *Xho*I restriction enzyme. Thus, in a more particular embodiment, the polynucleotide of the invention comprises the nucleotide sequence SEQ ID NO: 7.

### SEQ ID NO: 7 (the restriction sites are shown underlined)

A polynucleotide sequence may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression. The term "operably linked" has been previously defined in the present description.

Manipulation of the polynucleotide's sequence prior to its insertion into a vector may be desirable or necessary depending on the expression vector. The techniques for modifying polynucleotide sequences utilizing recombinant DNA methods are well known in the art. Thus, in another aspect, the present invention relates to a vector, hereinafter "vector of the invention", comprising the polynucleotide of the invention. Preferably, the vector is an expression vector. The term "expression vector" means a linear or circular DNA molecule that comprises a polynucleotide encoding a polypeptide and is operably linked to additional nucleotides (control sequences) that provide for its expression. The term "operably linked" has been previously defined.

The recombinant expression vector may be any vector (e.g., a plasmid or virus) that can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

Examples of bacteria vectors include, without limiting to, pUB110 (Muller et al. 1986, Mol.Gen.Genet, 202: 169-171), ColE1 (Chan et al. 1985. J. Biol. Chem. 260: 8925-8935), Ip25 (Casjens et al. 2000. Mol.Microbiol. 35: 490-516), pNOB8 (She et al. 1998. Extremophiles, 2: 417-425), F (Frost et al. 1994. Microbiol.Rev., 58: 162-210), SCP1 (Yamasaki, et al. 2000. J. Bacteriol., 182: 3140-3110) and pSymA (Barnett et al. 2001. Proc. NatI.Acad.Sci.USA, 98:9883-9888), pUC18 and pUC19 (NEB), pBluescript vector (Stratagene), pACYC vectors (NEB), Supercos (Stratagen), EMBL3 (Promega), λZAP (Stratagene) and pBeloBAC11 (NEB).

Examples of *E*. *coli* vectors include, without limiting to, pGEX (Amershan Biosciences), pEZZ (Amershan Biosciences), pPRO series (BD Biosciences Clontech), pHAT series (BD Biosciences Clontech), pBAD series (Invitrogen), pBAD/gIII A, B, C (Invitrogen), pBAD/Thio-TOPO (Invitrogen), pThioHis A, B, C (Invitrogen), pLEX (Invitrogen), Gateway pDEST (Invitrogen), pRSET A , B, C (Invitrogen), pCR-T7-TOPO (Invitrogen), pTreHis TOPO (Invitrogen), pTreHiS A, B, C (Invitrogen), lmpact^{™}CN series (New England BioLabs), pMALTMseries (New England BioLabs), pET series (Novagen), PinPointTM (Promega), pGEMEX (Promega), pQE series (Qiagen), pHB6 (Roche), pVB6 (Roche), pXB (Roche), pBX (Roche), pBH (Roche), pBV (Roche), pFLAG (Sigma) and pCAL series (Stratagene). In a particular embodiment, the expression vector is pET21a (EMD Biosciences; Bacterial expression vector with T7 lac promoter, adds N-terminal T7 epitope tag and C-terminal His tag; amp resistance; restriction enzyme cloning) and vectors derived from pNIC28-Bsa4. In another particular embodiment, the vector is PUC57 (GenScript, Township, NJ, USA).

Other useful vectors include, without limiting to, mammal cell-derived expression vectors (e.g., pcDNA3 (Invitrogen) and pEGF-BOS (Mizushima S., Nucleic Acids Res 18(17): 5322 (1990 )), pEF, pCDM8), insect cell-derived expression vectors (e.g., "Bac-to-BAC vaculovirus expression system" (GIBCO BRL), pBacPAK8), plant-derived expression vectors (e.g., pMH1, pMH2), animal virus-derived expression vectors (e.g., pHSV, pMV, pAdexLcw), retrovirus-derived expression vectors (e.g., pZlpneo), yeast-derived expression vectors (e.g., "Pichia expression kit" (Invitrogen), pNV11, SP-Q01), and Bacillus subtilisderived expression vectors (e.g., pPL608, pKTH50). Examples of vectors to express a polynucleotide in animal cells, such as CHO cells, COS cells or NIH3T3 cells, include, without limiting to, pMAM, pDR2, pBK-RSV, pBK-CMV, pOPRSV, or pOP13. Examples of vectors to express a polynucleotide in plant cells include, without limiting to, Ti plasmid from *Agrobacterium tumefaciens* and binary Ti vectors. Examples of the virus expression vectors include, without limiting to, attenuated virus hosts such as cowpox or avianpox, Bacille Calmette Guerin (BCG), adenovirus vectors and adeno - associated virus vectors, retrovirus vectors, *Salmonella typhi* vectors or detoxicated anthrax toxin vectors.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vectors preferably contain one or more (several) selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are the dal genes from *Bacillus subtilis* or *Bacillus licheniformis,* or markers that confer antibiotic resistance such as ampicillin, kanamycin, chloramphenicol, or tetracycline resistance. Suitable markers for yeast host cells are ADE2, HIS3, LEU2, LYS2, MET3, TRP1, and URA3. Selectable markers for use in a filamentous fungal host cell include, but are not limited to, amdS (acetamidase), argB (ornithine carbamoyltransferase), bar (phosphinothricin acetyltransferase), hph (hygromycin phosphotransferase), niaD (nitrate reductase), pyrG (orotidine-5'-phosphate decarboxylase), sC (sulfate adenyltransferase), and trpC (anthranilate synthase), as well as equivalents thereof.

The vectors preferably contain an element(s) that permits integration of the vector into the host cell's genome or autonomous replication of the vector in the cell independent of the genome.

For integration into the host cell genome, the vector may rely on the polynucleotide's sequence encoding the chimeric protein or any other element of the vector for integration into the genome by homologous or non-homologous recombination. Alternatively, the vector may contain additional nucleotide sequences for directing integration by homologous recombination into the genome of the host cell at a precise location(s) in the chromosome(s). The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" is defined herein as a nucleotide sequence that enables a plasmid or vector to replicate in vivo.

Examples of bacterial origins of replication are the origins of replication of plasmids pBR322, pUC19, pACYC177, and pACYC184 permitting replication in E. coli, and pUB1 10, pE194, pTA1060, and pAMβIpermitting replication in Bacillus.

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANSI (Gems et al., 1991, Gene 98: 61 -67; Cullen et al, 1987, Nucleic Acids Research 15: 9163-9175; WO 00/24883). Isolation of the AMA1 gene and construction of plasmids or vectors comprising the gene can be accomplished according to the methods disclosed in WO 00/24883.

More than one copy of a polynucleotide may be inserted into a host cell to increase production of the gene product. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors are well known to one skilled in the art.

In another aspect, the present invention relates to a host cell, hereinafter "cell of the invention", comprising the chimeric protein, the polynucleotide and/or the vector of the invention.

The term "host cell" means any cell type that is susceptible to transformation, transfection, transduction, and the like with a nucleic acid construct or expression vector comprising a polynucleotide of the present invention.

The present invention also relates to recombinant host cells, comprising the isolated polynucleotide of the invention, which are advantageously used in the recombinant production of the chimeric protein. A vector is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication.

The host cell may be any cell useful in the recombinant production of a polypeptide of the present invention, e.g., a prokaryote or a eukaryote such as a mammalian, insect, plant, or fungal cell.

The prokaryotic host cell may be any Gram positive bacterium or a Gram negative bacterium. Gram positive bacteria include, but not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but not limited to, *E. coli, Pseudomonas, Salmonella, Campylobacter, Helicobacter, Flavobacterium, Fusobacterium,* I*Iyobacter, Neisseria,* and *Ureaplasma.* In a particular embodiment, the host cell is *E. coli.* Examples of *E*. *coli* strains useful for vector propagation and cloning procedures include, without limiting to, DH10B (Invitrogen), DH5α (ATCC, Invitrogene), DM1 (Invitrogen), GeneHogs (Invitrogen), HB101 (Bio-Rad, Invitrogene, Promega), INV110 (Invitrogen), JM109 (ATCC, Promega), JS5 (Bio-Rad), LE392 (ATCC), NM522 (AS), SCS110 (Stratagene), STBL4 (Invitrogen), SURE (ATCC, Stratagene), TG1 (Stratagene), XL10-Gold (Stratagene) and XL1-Blue MRF (Stratagene). In a particular embodiment, the *E. coli* strain for cloning and storage is *E. coli* DH10B. In another particular embodiment, the *E*. *coli* strain for expressing the polynucleotide of the invention is *E. coli* BL21 (DE3) (EMD Biosciences, San Diego, CA).

Examples of the fungi host cells includes, without limiting to, Aspergillus (e.g. *Aspergillus awamori, Aspergillus fumigatus, Aspergillus foetidus, Aspergillus japonicus, Aspergillus nidulans, Aspergillus niger* or *Aspergillus oryzae* cell), Aureobasidium, Bjerkandera, Ceriporiopsis (e.g. *Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora),* Chrysosporium (e.g. *Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum),* Coprinus (e.g. *Coprinus cinereus),* Coriolus (e.g. *Coriolus hirsutus),* Cryptococcus, Filibasidium, Fusarium (e.g. *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell), Humicola (e.g. *Humicola insolens, Humicola lanuginosa),* Magnaporthe, Mucor (e.g. *Mucor miehei),* Myceliophthora (e.g. *Myceliophthora thermophila),* Neocallimastix, Neurospora (e.g. *Neurospora crassa),* Paecilomyces, Penicillium (e.g. *Penicillium purpurogenum),* Phanerochaete (e.g. *Phanerochaete chrysosporium),* Phlebia (e.g. *Phlebia radiata),* Piromyces, Pleurotus (e.g. *Pleurotus eryngii),* Schizophyllum, Talaromyces, Thermoascus, Thielavia (e.g. *Thielavia terrestris),* Tolypocladium, Trametes (e.g. *Trametes villosa or Trametes versicolor),* or Trichoderma (e.g. *Trichoderma harzianum, Trichoderma koningii, Trichoderma longibrachiatum, Trichoderma reesei, or Trichoderma viride* cell) cell.

The host cell may also be a yeast cell. "Yeast" as used herein includes ascosporogenous yeast (Endomycetales), basidiosporogenous yeast, and yeast belonging to the Fungi Imperfecti (Blastomycetes). More preferably, the yeast host cell is a Candida, Hansenula, Kluyveromyces (e.g. *Kluyveromyces lactis),* Pichia (e.g. *Pichia pastoris),* Saccharomyces (e.g. *Saccharomyces carlsbergensis, Saccharomyces cerevisiae, Saccharomyces diastaticus, Saccharomyces douglasii, Saccharomyces kluyveri, Saccharomyces norbensis, or Saccharomyces oviformis,* etc.), Schizosaccharomyces, or Yarrowia cell *(Yarrowia lipolytica).*

The host cell may also be microalgae. Examples of microalgae include, without limiting to, microalgae from the genera *Chlamydomonas* (e.g. *Chlamydomonas reinhardtii)* , *Botryococcus, Neochloris, Nannochloropsis, Phorphyridium, Scenedesmus, Chlorella, Tetraselmis* and *Spirulina.* The chloroplast of these algae are particularly well suited to the production of bacterial proteins as it mimics the native bacterial expression environment of these proteins while being devoid of their cell wall target.

The host cell may also be a plant cell, wherein the vacuolar deposition of recombinant proteins in plant vegetative organs is used to increase yields. To achieve this, the polynucleotide of the invention may be fused to sequence specific vacuolar sorting signals (ssVSS) typical of proteases or proteinase inhibitors and/or Ct-VSS representative of storage proteins or plant lectins. Both types of motifs were capable to increase accumulation. Importantly, the type of VSSs or position, either the N or C-terminus, did not alter protein stability, levels or post-translational modifications. Examples of plant cells include, without limiting to, *Nicotiana* sp. (e.g. *N. tabacum, N. benthamiana,* etc.) sugarcane, tomato *(Solanum lycopersicum)* and carrot *(Daucus carota).*

The introduction of DNA into a cell may, for instance, be effected by protoplast transformation, by using competent cells, by electroporation or by conjugation. Any method known in the art for introducing DNA into a host cell can be used.

In another aspect, the present invention relates to a composition, hereinafter "composition of the invention", comprising the chimeric protein, the polynucleotide, the vector or the cell of the invention.

According to the conventional techniques known to those skilled in the art, the composition according to the present invention may be formulated with excipient and/or carrier. Thus, in a particular embodiment, the composition of the invention comprises an excipient and/or carrier. In case of a therapeutic used of the composition of the invention, this may be formulated with a pharmaceutically acceptable excipient and/or carrier.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The term "carrier" refers to a compound which facilitates the incorporation of other compounds to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency. When the form of presentation is liquid, the carrier is the diluent. In a particular embodiment, the carrier is pharmaceutically acceptable.

The chimeric protein of the invention may be employed as a therapeutic agent to be administered to a subject. In this case, the composition provided by this invention is considered a pharmaceutical composition which may be administered by any appropriate administration route; to this end, said composition will be formulated in the suitable form for the selected administration route.

The "galenic form" or "pharmaceutical form" is the arrangement to which the active ingredients and excipients adapt in order to form a medicament. It is defined by the combination of the form in which the pharmaceutical composition is presented by the manufacturer and the form in which it is administered.

Routes of administration include topical, ocular, nasal, pulmonary, buccal, parenteral (intravenous, subcutaneous, and intramuscular), oral, vaginal and rectal. Administration from implants is also possible. The composition of the invention, in particular the pharmaceutical composition, may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated.

The composition of the invention may also be administered directly into the blood stream, into muscle, or into an internal organ. Suitable means for parenteral administration include intravenous, intra-arterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The composition of the invention may also be administered intranasally or orally. Dosage forms for oral administration include, without limiting to, tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms. The pharmaceutical composition may also be provided in bandages or in sutures or the like.

Many orthopedic surgeons consider that humans with prosthetic joints should be considered for antibiotic prophylaxis. Late deep infection by *S*. *aureus* is a serious complication sometimes leading to loss of the prosthetic joint and is accompanied by significant morbidity and mortality. It may therefore be possible to extend the use of the composition of the invention as a replacement for or for use in combination with prophylactic antibiotics in this situation. The composition of the invention may be administered by injection with a suitable carrier directly to the site of the orthopedic device *in situ* to clear the infection, or on a surface of the device prior to implantation.

Biodegradable particles (nanoparticles or microparticles) have been shown to be capable of delivering a variety of therapeutic agents including molecules such as polypeptides or proteins. Thus, the composition of the present invention also encompasses biodegradable microparticles o nanoparticles comprising the chimeric protein, the polynucleotide, the vector, the cell or the composition of the invention.

Additionally, the chimeric protein of the invention may be formulated as a pharmaceutically acceptable salt. The term "pharmaceutically acceptable salt" means a salt prepared from a base or an acid which is acceptable for administration to a patient, such as a mammal (e.g., salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a polypeptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (e.g.,citric, gluconic, glycolic, lactic, lactobionic, malic, and tartaric acids), aliphatic monocarboxylic acids (e.g., acetic, butyric, formic, propionic and trifluoroacetic acids), amino acids (e.g.,aspartic and glutamic acids), aromatic carboxylic acids (e.g.,benzoic, p-chlorobenzoic, diphenylacetic, gentisic, hippuric, and triphenylacetic acids), aromatic hydroxyl acids (e.g., o-hydroxybenzoic, p-hydroxybenzoic, 1-hydroxynaphthalene-2-carboxylic and 3-hydroxynaphthalene-2-carboxylic acids), ascorbic, dicarboxylic acids (e.g.,fumaric, maleic, oxalic and succinic acids), glucuronic, mandelic, mucic, nicotinic, orotic, pamoic, pantothenic, sulfonic acids (e.g., benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like.

The composition of the invention may comprise, further to the chimeric protein, the polynucleotide, the vector or the cell of the invention, other therapeutic agents such as antibiotics, painkillers, etc. Examples of antibiotics include, without limited to, vancomycin, teicoplanin, oxacillin, flucloxacillin, dicloxacillin, first generation cephalosporins (such as cefazolin, cephalothin, cephalexin, etc.), lincosamides (such as clindamycin, lincomycin, etc.), cotrimoxazole, erythromycin, rifampicin, fusidic acid, linezolid, quinupristin/dalfopristin and any combination thereof.

### Uses of the chimeric protein of the invention

As explained previously at the beginning of the present description, the chimeric protein of the invention has an improved lytic activity again *Staphylococcus* sp. and a higher stability than the parental endolysins of the state of the art. Thus, the chimeric protein of the invention shows killing activity against staphylococcal species, preferably, *S*. *aureus,* more preferably, *S*. *aureus* multi-drug resistant (MDR). Consequently, the chimeric protein of the invention, as well as all the aspects derived from it, may be effectively used as an antimicrobial not only in the treatment of infectious disease produced by *Staphylococcus* sp., particularly, *S*. *aureus,* more particularly, *S*. *aureus* multi-drug resistant, but also in the treatment of abiotic surfaces or food for disinfecting and/or decontaminating abiotic surfaces. Hereinafter, these inventive aspects are referred as the uses of the invention.

Consequently, in another aspect, the present invention relates to the chimeric protein, the polynucleotide, the vector, the cell or the composition of the invention for use as a medicament.

In another aspect, the invention relates to the chimeric protein, the polynucleotide, the vector, the cell or the composition of the invention for use in the treatment and/or prevention of an infection of *Staphylococcus* sp, preferably, *S*. *aureus,* more preferably, *S*. *aureus* multi-drug resistant (MDR).

The term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders associated with an infection of *Staphylococcus* sp, preferably, *S*. *aureus,* more preferably, *S*. *aureus* multi-drug resistant (MDR). The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. It is to be understood that a subject/patient may experience a broad range of responses to a treatment (such as the exemplary responses as described herein above). The treatment of associated with an infection of *Staphylococcus* sp, may, *inter alia,* comprise curative treatment (preferably leading to a complete response and eventually to healing of the disorder or disease) and palliative treatment (including symptomatic relief).

As used herein, the term "prevention" refers to the inhibition or delay of an infection of *Staphylococcus* sp, preferably, *S*. *aureus,* more preferably, *S*. *aureus* multi-drug resistant (MDR) in a subject.

Examples of *Staphylococcus* species includes, without limited to, *S*. *argenteus, S. argensis, S. aureus, S. schweitzeri, S. simiae, S. auricularis, S. carnosus, S. caseolyticus, S. condimenti, S. massiliensis, S. piscifermentans, S. simulans, S. capitis, S. caprae, S. epidermidis, S. saccharolyticus, S. devriesei, S. haemolyticus, S. hominis, S. agnetis, S. chromogenes, S. felis, S. delphini, S. hyicus, S. intermedius, S. lutrae, S. microti, S. muscae, S. petrasii, S. pettenkoferi, S. pseudintermedius, S. pulvereri, S. rostri, S. schleiferi, S. lugdunensis, S. arlettae, S. cohnii, S. equorum, S. gallinarum, S. kloosii, S. nepalensis, S. saprophyticus, S. succinus, S. xylosus, S. fleurettii, S. lentus, S. sciuri, S. stepanovicii, S. vitulinus, S. pasteuriand S. warneri.* In a particular embodiment, the disease is produced by an infection of *S*. *aureus,* more in particular, *S*. *aureus* MDR.

*S*. *aureus* (also known as golden staph) is a gram-positive, round-shaped bacterium that is a member of the Firmicutes, and is frequently found in the nose, respiratory tract, and on the skin and mucose membranes of humans and some other animals.

*S*. *aureus* can cause a range of illnesses, from minor skin infections to life-threatening diseases. Examples of these include, without limiting to, pimples, impetigo, boils, cellulitis, folliculitis, carbuncles, scalded skin syndrome, abscesses, pneumonia, meningitis, osteomyelitis, endocarditis, toxic shock syndrome, bacteremia, and sepsis.

The subject or patient to be treated of a disease related to a *Staphylococcus* sp, preferably S. *aureus,* infection, such as the subject in need of treatment, may be an animal (e.g., a non-human animal), a vertebrate animal, a mammal, a rodent (e.g., a guinea pig, a hamster, a rat, a mouse), a canine (e.g., a dog), a feline (e.g., a cat), a porcine (e.g., a pig), an equine (e.g., a horse), a primate, a simian (e.g., a monkey or ape), a monkey (e.g., a marmoset, a baboon), an ape (e.g., a gorilla, chimpanzee, orangutan, gibbon), or a human. In the context of this invention, it is also envisaged that animals are to be treated which are economically or agronomically important. Non-limiting examples of agronomically important animals are sheep, cattle and pigs, while, for example, cats and dogs may be considered as economically important animals. Preferably, the subject/patient is a mammal; more preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig); most preferably, the subject/patient is a human.

Particularly, the human subject may be an infant, a toddler, an adolescent, a teenager, or an adult (e.g., at least 18 years old, at least 20 years old, at least 25 years old, at least 30 years old, at least 35 years old, at least 40 years old, at least 45 years old, at least 50 years old, at least 55 years old, at least 60 years old, at least 65 years old, at least 70 years old, at least 75 years old, at least 80 years old, at least 85 years old, at least 90 years old, at least 95 years old, or at least 100 years old). In some examples, the subject has a suppressed or weakened immune system (e.g., humoral or cellular immune system).

The polypeptide, the polynucleotide, the vector, the cell, or the composition of the invention can be administered to the subject suffering from an infection of *Staphylococcus* sp, preferably, *S*. *aureus,* more preferably, *S*. *aureus* multi-drug resistant (MDR) in combination with a second therapeutic agent, wherein the second therapeutic agent is an antibiotic agent or immunotherapeutic agent. Exemplary antibiotic agents include, without limitation, linezolid, erythromycin, mupirocin, ertapenem, doripenem, imipenem, cilastatin, meropenem, cefadroxil, cefazolin, cefalotin, cefalothin, cephalexin, ceflacor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, ceftaroline fosamil, ceftobiprole, teicoplanin, vancomycin, televancin, clindamycin, lincomycin, daptomycin, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, penicillin G, temocillin, ticarcillin, bacitracin, colistin, polymyxin B, ciprofloxacin, enoxacin, gatifloxacin, gemifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sufamethizole, sulfamethoxazole, sulfanilamide, sulfasalazine, sulfisoxazole, trimethoprim-sulfamethoxazole, sulfonamidochrysoidine, demeclocycline, doxycycline, minocycline, oxytetracycline, tetracycline and combinations thereof. Exemplary immunotherapeutic agents include, without limitation, tefibazumab, BSYX-A110, Aurexis^{™}, and combinations thereof. The above lists of antibiotic agents and immunotherapeutic agents are intended to be non-limiting examples; thus, other antibiotic agents or immunotherapeutic agents are also contemplated. Combinations or mixtures of the second therapeutic agent can also be used for the purposes of the present invention. These agents can be administered contemporaneously or as a single formulation.

In order to treat a disease, i.e. a disease associated to *Staphylococcus* sp, preferably, *S*. *aureus,* more preferably, *S*. *aureus* multi-drug resistant (MDR), the chimeric protein of the invention (as well as all the aspects derived from it such as the polynucleotide, the vector, the cell or the composition of the invention) is administered in an "effective dose" to the subject to be treated. As used herein, the term "effective amount" or "effective dose" or "pharmaceutically effective amount" refers to a quantity sufficient to achieve a desired therapeutic effect, e.g. an amount which results in the treatment of, or a decrease in, an infection of *Staphylococcus* sp. In the context of therapeutic applications, the amount of a composition administered to the subject will depend on the type and severity of the disease and on the characteristics of the individual, such as general health, age, sex, body weight and tolerance to drugs. It will also depend on the degree, severity and type of infection. The skilled artisan will be able to determine appropriate dosages depending on these and other factors. The precise dose will ultimately be at the discretion of the attendant physician or veterinarian.

When *in vivo* administration of the chimeric protein of the invention (as well as all the aspects derived from it) is employed, normal dosage amounts may vary from about 10 ng/kg to up to 1000 mg/kg of mammal body weight or more per day, or about 1 µg/kg/day to 10000 mg/kg/day, depending upon the route of administration. Guidance about particular dosages and methods of delivery is also provided below as well as in the literature. It is anticipated that different formulations will be effective for different treatment compounds and different disorders. The administration targeting a particular organ or tissue may require different delivery than another organ or tissue. The chimeric protein of the invention may be applied anywhere from once to several times a day, and may be applied for a short or long term period. The usage may last for days or weeks. Any dosage form employed should provide for a minimum number of units for a minimum amount of time. The concentration of the active units of a chimeric peptide believed to provide for an effective amount or dosage of enzyme may be in the range of about 10, 20, 30, 40, 50, 60, 70, 80, 90, or 100 units/mL up to about 10,000,000 units/ml of composition, in a range of about 1000 units/mL to about 10,000,000 units/mL, and from about 10,000 to 10,000,000 units/mL.

The compositions can also be administered in combination with one or more additional therapeutic compounds for the treatment of infections of *Staphylococcus* sp, preferably, *Staphylococcus aureus,* more preferably, *S*. *aureus* multi-drug resistant (MDR). Examples of therapeutic compounds have been disclosed in the above paragraphs.

Methods of treatments equivalent to the uses of the present invention are also encompassed within the inventive aspects of the present invention. Thus, the invention relates to a method for the treatment of a subject from an *S*. *aureus* infection comprising administering to the subject the chimeric protein, the polynucleotide, the vector, the cell or the composition of the invention. The term "subject" and "treatment" have been previously disclosed in the present description.

As it is known, bacteria persisting in a biofilm in the mammalian body cause about twothirds of all chronic or recurrent diseases. These biofilms are comprised of bacteria protected by an outer "slime" that is often comprised primarily of DNA that prevents the innate and adaptive immune systems, antibiotics and other antibacterial agents from gaining access to the bacteria inside the biofilm, making it extremely difficult to clear the infection from the body. Furthermore, the biofilm can act as a reservoir for future acute infections often with lethal consequences. Thus, biofilm infections represent a clinical challenge, as they are highly resistant to antimicrobial therapies and often occur in areas of the body that are not easily accessible for treatment. Staphylococci, in particular, represent a large portion of biofilm-based infections such as chronic wound infection, chronic otitis media, chronic osteomyelitis, chronic rhinosinusitis, recurrent urinary tract infection, endocarditis and cystic fibrosis-associated lung infection among others. Moreover, they also cause infections of medical devices and surgical wounds, being a significant burden on the healthcare system.

The term "biofilm" refers to an organized community of microorganisms that at times adhere to the surface of a structure that may be organic or inorganic, together with the polysaccharides, proteins and DNA that they secrete and/or release. The biofilms are very resistant to microbiotics and antimicrobial agents.

The present invention also encompasses the uses of the chimeric protein, the polynucleotide, the vector the host or the composition of the invention for inhibiting, preventing or breaking down a microbial biofilm comprising *Staphylococcus* sp, preferably, S. *aureus,* more preferably, *S*. *aureus* multi-drug resistant (MDR). Therefore, the present invention also provides a method of inhibiting, preventing or breaking down a biofilm in a subject comprising administering to the subject an effective amount of the chimeric protein, the polynucleotide, the vector the host or the composition of the invention, thereby inhibiting, preventing, removing or breaking down the microbial biofilm.

Additionally, due to the capacity of the chimeric protein of the invention (as well as all the aspects derived from it) of killing *Staphylococcus* sp., its *ex vivo* use as an antimicrobial is also contemplated within the scope of the present invention. Examples of *ex vivo* uses include, without limiting to, cleaning or disinfecting surfaces in the food industry, in farms (such as stables, barns, etc), in laboratories and in hospitals (such as rooms, operating rooms, surgery tools, floors invasive medical devises (catheters, valves, prosthesis, etc). The formation of biofilms can also occur in industrial settings since it is the cause of operating troubles by decreasing heat transfer, blocking tubes, plugging filters and causing damage to surfaces. Specifically in the food industry, the ability of bacteria to attach to foodcontact surfaces provides a reservoir of contamination for pathogens with the consequent risks to human health. Analysis of the microbial composition of biofilms formed on food industrial surfaces revealed the presence of mixed biofilms including pathogenic and spoilage bacteria. These microorganisms can reach the food industry through several sources such as water, raw foods, animals, handlers, and can persist in the equipment for long periods of time. Therefore, food products can be contaminated at any stage of the food chain, even though all required cleaning protocols have been applied because disinfecting and cleaning processes in the food industry are often ineffective. For instance, in the dairy industry, some microorganisms are able to survive after cleaning-in-place (CIP) procedures. Biofilms mainly cause problems in dairy, meat, poultry, seafood and vegetable processing industries. Depending on the industry, the route of access to foodstuffs differs.

In view of the above, other aspect of the present invention relates to the use of the chimeric protein, the polynucleotide, the vector, the host cell and/or a composition of the invention for removing, inhibiting and/or preventing *Staphylococcus* sp. biofilm formation on abiotic surfaces. The term biofilm, and examples thereof, has been defined above.

In another aspect, the invention relates to the use of the chimeric protein, the polynucleotide, the vector, the host cell and/or a composition of the invention for removing and/or preventing the *Staphylococcus* sp. contamination of foods and/or abiotic surfaces.

As use herein, the term "removing" refers to reduce the *Staphylococcus* sp. load up to an amount which is considered no harmful for a subject, preferably, a human subject. As the skilled person knows, the microbial load which can be present in a food (or in an abiotic surface) depends on the kind of food and the microorganism. The minimal microbial load allowed being present in foods (or abiotic surfaces) for each pathogen microorganism is widely known in the state of the art and can be checked in international manuals.

In the case of *Staphylococcus* sp., examples of minimal microbial load include, without limited to, the following values:
- In cheeses made with raw milk the limit of coagulase-positive staphylococci is 10⁴ - 10⁵ CFU/g (Analytical reference method: EN/ISO 6888-2).
- In cheeses made from milk that has undergone a lower heat treatment than pasteurization and in ripened cheeses made from milk or whey that has undergone pasteurisation or a stronger heat treatment, the limit is 100 - 1000 CFU/g (Analytical reference method: EN/ISO 6888-1 or 2);
- In unripened soft cheeses (fresh cheeses) made from milk or whey that has undergone pasteurisation or a stronger heat treatment the limit is 10 - 100 CFU/g (Analytical reference method: EN/ISO 6888-1 or 2).
- Likewise, staphylococcal enterotoxins should not be detected in 25 g of cheese, milk powder and whey powder products (Analytical reference method: European screening method of the CRL for coagulase positive staphylococci).
- In shelled and shucked products of cooked crustaceans and molluscan shellfish, the limit of coagulase-positive staphylococci is 100 -1000 CFU/g (Analytical reference method: EN/ISO 6888-1 or 2)

These data have been obtained from COMMISSION REGULATION (EC) No 2073/2005 of 15 November 2005 on microbiological criteria for foodstuffs and COMMISSION REGULATION (EC) No 1441/2007 of 5 December 2007 amending Regulation (EC) No 2073/2005 on microbiological criteria for foodstuffs.

As use herein, in the context of the present inventive aspect, the term "preventing" or "prevent" refers to prevent, to stop or to impede a surface or a food from being contaminated or colonized by *Staphylococcus* sp. Thus, the chimeric protein of the invention (and all the aspects derived from it) can be used as bio-conservative in food products and as a disinfectant in food and agricultural settings.

Examples of foods which can be treated with the chimeric protein, the polynucleotide, the vector, the host cell and/or a composition of the invention include, without limiting to, animal feed, dairy products, vegetable products, meat products, snacks, chocolates, drinks, baby food, cereals, fried foods, bakery products, cookies, etc. Examples of dairy products include, but are not limited to, fermented milk products (such as, but not limited to, yogurt or cheese) or non-fermented products (for example, but not limited to, cream, butter, margarine or whey). A vegetable product is, for example, but not limited to, a cereal in any form of presentation, fermented or unfermented, or a snack. The beverage may be, but is not limited to, non-fermented milk.

In the context of the present invention, the term "abiotic surface" refers to any inert surface made of any material. Examples to materials include, without limiting to, polystyrene, stainless steel, rubber, ceramic, polycarbonate, plastic, glass, metal and any other material used for medical devices manufacturing.

Examples of places whose abiotic surfaces may be treated with the chimeric protein, the polynucleotide, the vector, the host cell and/or a composition of the invention include, without limiting to, stables, barns, laboratories and hospitals (such as rooms, operating rooms, surgery tools, floors invasive medical devises (catheters, valves, prosthesis, etc), tubes, pipes and plugging filters.

In a particular embodiment, the *Staphylococcus* sp. is *Staphylococcus aureus,* more preferably, S. *aureus* multi-drug resistant (MDR)

In another aspect, the present invention relates to a kit, hereinafter "kit of the invention", comprising the chimeric protein, the polynucleotide, the vector, the host cell and/or the composition of the invention.

In the present invention, a "kit" is understood as a product comprising the different reagents for the treatment of *Staphylococcus* sp, preferably, *S*. *aureus,* more preferably, *S*. *aureus* multi-drug resistant (MDR) infection, i.e. the chimeric protein, the polynucleotide, the vector, the cell, or the composition of the invention. Another component which can be present in the kit is a packing which allows maintaining the reagents within determined limits. Suitable materials for preparing such packings include glass, plastic (polyethylene, polypropylene, polycarbonate and the like), bottles, vials, paper, sachets and the like. The kit of the invention can additionally contain instructions for using the reagents in the method of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

Therefore, the present invention also encompasses the use of the the kit of the invention for treating a subject from a *Staphylococcus* sp, preferably, *S*. *aureus,* more preferably, S. *aureus* multi-drug resistant (MDR) infection.

The present invention also relates to methods of producing the chimeric protein of the present invention comprising cultivating a recombinant host cell, as described herein, under conditions conducive to producing the polypeptide of the invention and recovering said polypeptide. Thus, in another aspect, the present invention relates to a method for producing a chimeric protein of the invention, hereinafter "method of the invention", comprising
(i) transforming a host cell with the polynucleotide or the vector of the invention,
(ii) culturing the host cell resulting from step (i) under conditions for expressing the polynucleotide, or the vector of the invention and optionally,
(iii) recovering the chimeric protein.

Methods for transforming or transfecting host cells with polynucleotides or vectors are well-known in the art and depend on the host system selected, as described in Sambrook & Russell, Molecular Cloning: a laboratory manual (Cold Springs Laboratory Press, 2001). For bacterial cells, suitable techniques include calcium chloride transformation, electroporation, and transfection using bacteriophage. For eukaryotic cells, suitable techniques include calcium phosphate transfection, DEAE-Dextran, electroporation, liposome-mediated transfection, and transduction using retrovirus or any other viral vector. For insect cells, the transfer vector containing the polynucleotide construct of the present invention is co-transfected with baculovirus DNA, such as AcNPV, to facilitate the production of a recombinant virus. Subsequent recombinant viral infection of Sfcells results in a high rate of recombinant protein production.

In the production method of the present invention, the cells are cultivated in a nutrient medium suitable for production of the enzyme composition using methods well known in the art. For example, the cell may be cultivated by shake flask cultivation, and small-scale or large-scale fermentation (including continuous, batch, fed-batch, or solid state fermentations) in laboratory or industrial fermenters performed in a suitable medium and under conditions allowing the polynucleotide to be expressed and/or the resulting protein isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the chimeric protein is secreted into the nutrient medium, the enzyme composition can be recovered directly from the medium. If the chimeric protein is not secreted into the medium, it can be recovered from cell lysates.

Once the polypeptide is produced by the culturing of the host cell, it may be detected, purified and/or isolated using methods known in the art. These detection methods include, without limiting to, the use of specific antibodies, formation of an enzyme product, or disappearance of an enzyme substrate. The chimeric protein may be recovered by conventional procedures including, but not limited to, ion affinity chromatography, centrifugation, filtration, extraction, spray-drying, evaporation, or precipitation.

As used herein the term "purified" in the expression "purified chimeric protein" means altered "by the hand of man" from its natural state (i.e. if it occurs in nature, it has been changed or removed from its original environment) or it has been synthesized in a non-natural environment. These terms do not require absolute purity (such as a homogeneous preparation) but instead represents an indication that it is relatively more pure than in the natural environment.

Alternatively, the chimeric protein of the invention can also be obtained by synthesis. Particular techniques for synthesizing peptides or proteins include classical methods in which peptides of increasing size are isolated before each amino acid or preformed peptide addition, classical chemical synthesis amino acid by amino acid, and solid phase peptide synthesis in which the peptide is built up attached to a resin such as a Merrifield resin. In these synthetic procedures, groups on the amino acids will generally be in a protected from using standard protecting groups such as t-butoxycarbonyl. If necessary, these protecting groups are cleaved once the synthesis is complete. Chemistry synthesis methods - for example by peptide synthesis in solid phase, solution synthesis, a combination of methods of solid phase synthesis and solution or enzymatic synthesis - are known by the expert in the field.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

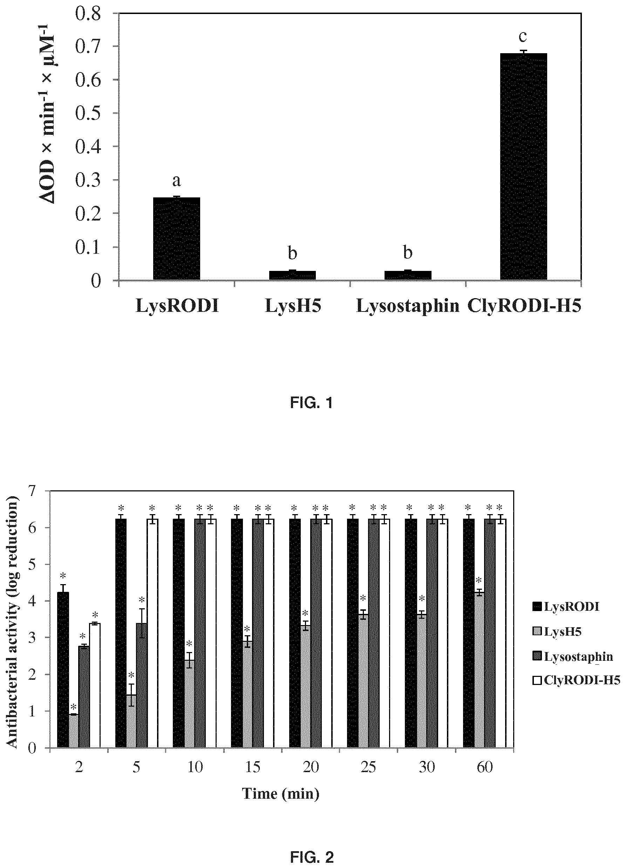

**Figure 1****.** Specific lytic activity of the proteins against *S*. *aureus* Sa9. Bars represent the activity (ΔDO600 × minutes⁻¹ × µM⁻¹) of each protein. Data are the mean ± standard deviation of three biological replicates. Bars having distinct lower case letter are statistically different (p<0.05) according to the ANOVA and SLK post-hoc comparison test.
**Figure 2****.** Time-kill curve of S. *aureus* Sa9 treated with equimolar amounts of proteins (0.1 µM). Results (means ± standard deviations of three replicates) are reported as bacterial reduction (Iog10 units) relative to the untreated control. Bars having an asterisk are statistically different (p<0.05) from the untreated control, according to the Student t-test.

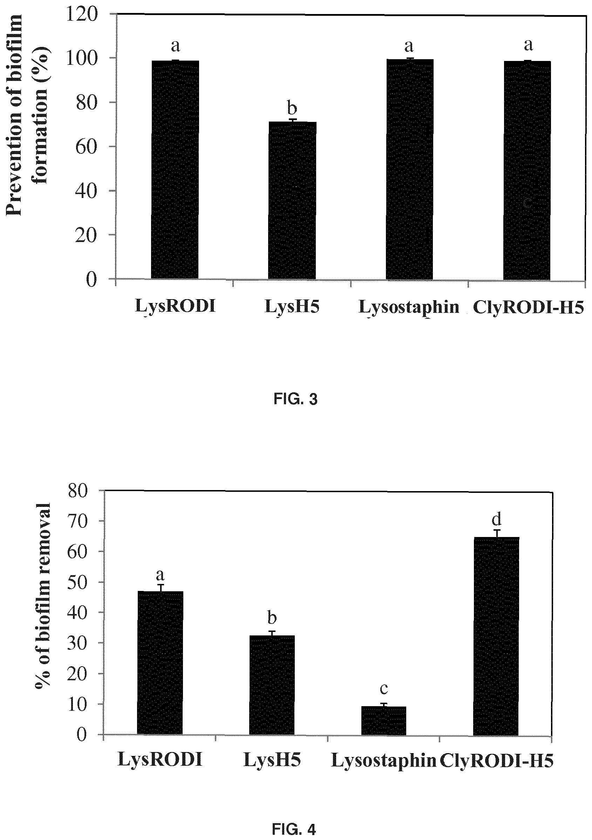

**Figure 3****.** Biofilm growth inhibition in the presence of 0.7 µM of protein. Percentage of biofilm inhibition was calculated by crystal violet staining after growth of biofilms for 24 hours. Results are reported as means ± standard deviations of three replicates. Bars having distinct lower case letter are statistically different (p<0.05) according to the ANOVA and SLK post-hoc comparison test.
**Figure 4****.** Removal of 8 hours S. *aureus* 15981 biofilms after addition of 2.5 µM of protein and further incubation at 37ºC for 16 h. The percentage of biofilm elimination was calculated by crystal violet staining after the treatment. Results are reported means ± standard deviations of three replicates. Bars having distinct lower case letter are statistically different (p<0.05) according to the ANOVA and SLK post-hoc comparison test.

### Example 1

### Design of a chimeric-phage protein with high antimicrobial activity against Staphylococcus aureus MRSA

### I. MATERIAL AND METHODS

### S. aureus endolysins.

Parental endolysins are encoded in two bacteriophages: philPLA88 (a lytic derivative of the temperate phage ØH5) (42,526 bp; 61 ORF) (Garcia, P., et al. 2009a. J Dairy Sci. 92, 3019-3026; and Garcia, P., et al. 2009b. Appl Environ Microbiol. 75, 7663-7673); and philPLA-RODI (142,348 bp; 213 ORF) (Gutiérrez et al., 2015a. Appl Environ Microbiol. 81, 3336-3348). The genes encoding the endolysins were identified into the phage genomes: Endolysin LysH5 (481 amino acids; 54.2 kDa) is encoded by orf61 (1,446 nucleotides) of phage philPLA88 genome. This protein has been previously characterized (Obeso et al., 2008, Int J Food Microbiol. 128, 212-218.). Endolysin LysRODI (496 amino acids, 54.81 kDa) is encoded by orf57 (1,491 nucleotides) of phage phiIPLA-RODI genome.

### Bacterial strains and culture conditions.

All the staphylococcal strains (Table 1) used in this study were grown in tryptic soy broth (TSB; Difco, Franklin Lakes, NJ) at 37°C with shaking or on TSB plates containing 2% (wt/vol) bacteriological agar (TSA). *Escherichia coli* DH10B (Invitrogen, Carlsbad, CA) was used for cloning and storage. *E. coli* BL21 (DE3) (EMD Biosciences, San Diego, CA) was used for protein expression. All *E. coli* strains were grown at 37ºC with shaking in LB medium (1% tryptone, 0.5% yeast extract, 1% NaCl) or in LB supplemented with 2% (w/v) agar. For proper selection of the clones, 100 µg/ml ampicillin or 50 µg/ml kanamycin were used.

| **Specie** | **Strain** | **Origin** | **Ref.** | **MIC (µM)** | | | |
|---|---|---|---|---|---|---|---|
| | | | | **LysRODI** | **LysH5** | **ClyRODI-H5** | **Lysostaphin** |
| *S. aureus* | **Sa9** | Cow milk | (1) | 0.57 | 3.83 | 1.84 | 0.43 |
| | **15981** | Clinical isolate | (2) | 1.15 | 3.83 | 1.84 | 0.43 |
| MRSA | **7829** | Clinical isolate | (3) | 1.15 | 3.83 | 1.84 | 0.86 |
| | **2016-19142-2** | | | 1.15 | 3.83 | 1.84 | 0.43 |
| | **H1 9/3** | | | 1.15 | 3.83 | 1.84 | 0.43 |
| | **Staph. 10** | | | 1.15 | 3.83 | 1.84 | 0.43 |
| | **Staph. 11** | | | 1.15 | 3.83 | 1.84 | 0.86 |
| | **Staph. 15** | | | 1.15 | 3.83 | 1.84 | 0.43 |
| | **E10** | Pig skin (ear) | (4) | 1.15 | 3.83 | 1.84 | 0.43 |
| *S. epidermidis* | **F12** | Women breast milk | (5) | 1.15 | 7.65 | 7.37 | 1.72 |
| | **b** | | | 0.57 | > 15.31 | >14.76 | 0.86 |
| | **DG2Ñ** | | | 1.15 | > 15.31 | >14.76 | 3.45 |
| | **LO5081** | | | 1.15 | >15.31 | >14.76 | 1.72 |
| | **LV5RB3** | | | 2.29 | >15.31 | >14.76 | 1.72 |
| *S. sciuri* | **101** | Dairy industry surface | (6) | 4.59 | >15.31 | >14.76 | 1.72 |
| *S. hominis* | **ZL31-13** | Women breast milk | (7) | 1.15 | >15.31 | >14.76 | 3.45 |
| *S. pasteuri* | **ZL16-6** | | | 2.29 | >15.31 | >14.76 | 0.43 |
| S. *xylosus* | **ZL61-2** | | | 1.15 | >15.31 | >14.76 | 0.86 |
| *S. saprophyticus* | **ZL112-15** | | | 0.15 | > 15.31 | >14.76 | 1.72 |
| *S. arlettae* | **ZL114-5** | | | 1.15 | > 15.31 | >14.76 | 1.72 |
| *S. haemolyticus* | **ZL89-3** | | | 0.57 | 7.65 | 3.68 | >13.80 |
| *S. gallinarum* | **ZL90-5** | | | 1.15 | >15.31 | >14.76 | 6.90 |
| *S. kloosii* | **ZL74-2** | | | 1.15 | >15.31 | >14.76 | 0.86 |

**Table 1.** Staphylococcal strains used in this study, origin, and sensitivity to lytic proteins expressed as minimum inhibitory concentration (MIC). MIC is expressed as the mode of three independent biological repeats (µM). (Ref.) Reference; (1) Garcia et al., 2007 Int Dairy J. 17, 7.; (2) Valle et al., 2003, Mol Microbiol. 48, 1075-1087.; (3), (4), (6) Unpublished; (5) Delgado et al., 2009. BMC Microbiol. 9, 82; (7) Martin et al., 2012 J Hum Lact. 28, 36-44.

### Plasmid construction and DNA manipulation.

The genes encoding LysRODI (Genbank accession number YP_009195893.1, SEQ ID NO: 7) and LysH5 (Genbank accession number YP_002332536.1, SEQ ID NO: 8) from phages vB_SauM_philPLA-RODI and vB_SauS-phiIPLA88, respectively, were optimized based on the *E. coli* codon usage by the OptimumGeneTM codon optimization technology. Additionally, Ndel and Xhol restriction sites were added at 5' and 3' terminal sites. The optimized sequences were synthetized, cloned into pUC57 vector by GenScript (Township, NJ, USA), and sub-cloned into the expression vector pET21a that introduces a C-terminal His6-tag and carries an ampicillin resistance gene.

For chimeric protein construction, the conserved domains from LysRODI and LysH5 were predicted from the protein sequence using BLASTP (Altschul et al., 1990. J Mol Biol. 215:403-410.) and HMMER v3.1b2 (hmmer.org). The predicted 3D structure was calculated using Phyre2. The in silico design of the chimeric protein ClyRODI-H5 was constructed by the fusion of the CHAP domain from LysRODI and the SH3b domain from LysH5. In order to ensure proper folding, the CHAP domain from LysRODI was cloned from the N-terminus of the protein and flanked by additional 5 amino acids after the C-terminus. In a similar way, the SH3b domain from LysH5 was cloned from 56 amino acids located before the N-terminus of this domain to the C-terminus of the protein. The gene encoding ClyRODI-H5 was obtained, at the Laboratory of Gene Technology, Faculty of Bioscience Engineering (University of Leuven, Belgium), using the VersaTile Shuffling technique (currently under patent), followed by a sub-cloning into an expression vector derived from pNIC28-Bsa4 that carries a kanamycin resistance gene.

The expression vectors containing the endolysins and the chimeric protein genes were finally transformed into E. coli BL21 (DE3) by electroporation.

### Protein overexpression and purification.

Protein purification was performed as previously described with some modifications (Gutiérrez et al., 2015b, Front Microbiol. 6, 1315). Briefly, exponentially growing cultures (OD600 = 0.5) were induced with 1 mM IPTG (isopropyl-β-D-thiogalactopyranoside) and incubated at 16°C for 16 hours. Five hundred milliliter culture cells were pelleted (10,000 rpm, 30 minutes), suspended in 10 ml lysis buffer (20 mM NaH2PO4, 500 mM NaCl, 10 mM imidazole, pH 7.4) and frozen/thawed three times at -80°C. Sonication was carried out afterwards (15 × 5 s pulses with 15 s recovery on ice). Then, the suspension was centrifuged at 10,000 rpm. Each His6-tagged fusion protein was purified from the cleared supernatant by immobilized metal ion affinity chromatography using nickel-NTA Superflow resin columns (Qiagen, Valencia, CA, USA). For purification of bound proteins, the washing and elution profiles were empirically determined to be 20 mL of 10 mM imidazole, 40 ml of 20 mM imidazole and elution with 1 ml of 250 mM imidazole in phosphate buffered saline (50 mM NaH2PO4, 300 mM NaCl, pH 7.4). Purified proteins were stored at -80ºC with 30% glycerol to prevent precipitation. The purity of proteins was evaluated in 12% (vol/wt) SDS-PAGE run at 150 V using Criterion precast gels (Bio-Rad, Inc., Hercules, CA), and further revealed via conventional Coomassie staining. Prior to performing activity experiments, all purified proteins were filtered using 0.45 µm PES membrane filters (VWR, Spain) and diluted into 50 mM sodium phosphate buffer (pH=7.4). Protein amount was quantified by the Quick Start Bradford Protein assay (BioRad, Hercules, CA, USA).

### Quantification of specific lytic activity.

Turbidity reduction assay was performed as previously described (Obeso et al., 2008. Int J Food Microbiol. 128, 212-218.) using *S*. *aureus* Sa9 cells suspended in 50 mM sodium phosphate buffer, (pH=7.4) and treated with two fold dilutions of the purified proteins (0.02-50 µM). The activity of the bacteriocin Lysostaphin (Sigma, Missouri, USA), produced by Staphylococcus simulans, was also tested as positive control. Results were expressed as the specific lytic activity (ΔOD6OO × minutes⁻¹ × µM⁻¹). To evaluate the activity of the proteins in the presence of different ions (KCI, MgCI2, NaCl, MnCI2, ZnCI2, CaCl2), these were added to a final concentration of 1 mM to the turbidity assay. The stability of the proteins to different physicochemical conditions was evaluated by incubating an aliquot of the purified protein for 30 minutes at temperatures ranging from 40°C to 90°C. Afterwards, the turbidity reduction assay was carried out as described above. Moreover, the activity of the proteins was tested by diluting (1:100) each purified protein into the Britton-Robinson pH universal buffer (150 mM KCI, 10 mM KH2PO4, 10 mM sodium citrate, 10 mM H₃BO₃) adjusted to a pH from 3 to 11. In this case, the turbidity reduction assay was carried out with *S*. *aureus* Sa9 cells suspended in the Britton-Robinson buffer. All experiments were performed using three biological replicates.

### Minimum inhibitory concentration (MIC) of phage lytic proteins.

The MIC of the proteins was determined in triplicate by a conventional broth microdilution technique in TSB (CLSI, 2015). Two-fold dilution of each protein was added to a microtiter plate, containing each well 106 CFU of staphylococcal strain. The MIC was defined as the lowest protein concentration that inhibited visible bacterial growth after 24 hours of incubation at 37°C, and it was expressed as the mode of three independent biological repeats.

### Time killing assay.

Exponentially growing cells (OD600 = 0.5) of *S*. *aureus* Sa9 in TSB medium were harvested by centrifugation (10,000 rpm, 5 minutes), washed twice and suspended in 50 mM sodium phosphate buffer (pH=7.4) to a final concentration of ± 106 CFU/mL. Each protein (0.1 µM) was added to 1 ml of bacterial suspension and incubated at 37°C with shaking. At different time points (2 - 60 minutes), 50 µL were taken, and 0.15 µg of Proteinase K were added to stop the reaction. Appropriate dilutions of the suspensions were plated onto TSB agar and incubated at 37ºC for 16 hours. The antibacterial activity was quantified as the relative inactivation in log units (Iog10 [N0/Ni] with N0 as the initial number of untreated cells and Ni as the number of residual cells counted after treatment). All the experiments were performed using three biological replicates.

### Determination of bacterial resistance to lytic proteins.

Resistance development was tested using repeated exposures calculated according to the MIC values (Rodriguez-Rubio et al., 2013. PLoS One, 8, e64671). Two-fold serial dilutions of the proteins (0.02 - 50 µM) were used against *S*. *aureus* Sa9 (106 CFU) grown in a 96 polystyrene microtiter plate, at 37ºC for 16 hours. The next day, 100 µl of the first well with growth (1/2 MIC) were inoculated into 5 mL of TSB and grown to an OD600=0.5. These cultures were used for the next round of MIC exposure. The experiment was repeated for 10 rounds, followed by 5 additional rounds in TSB without the protein in order to allow phenotype reversion. MIC assay was further performed to measure the sensitivity to the proteins. Experiments were performed using three independent biological replicates.

### Biofilm assays.

Staphylococcal biofilms were obtained as previously described (Gutiérrez et al., 2014 PLoS One. 9, e107307) using *S*. *aureus* 15981. Briefly, overnight (o/n) cultures were diluted in fresh TSBg (TSB supplemented with 0.25% w/v D-(+)-glucose) up to 106 CFU/mL, and 200 µL were poured into TC Microwell 96U w/lid nunclon D SI plates (Thermo Scientific, NUNC, Madrid, Spain), and incubated at 37°C for 8 hours. Wells were washed twice with sterile phosphate-buffered saline (PBS buffer) (137 mM NaCl, 2.7 mM KCI, 10 mM Na2HPO4 and 2 mM KH2PO4; pH 7.4). For treatment of staphylococcal biofilms, 200 µL of each protein (2.5 µM final concentration) or 50 mM sodium phosphate buffer, (pH 7.4) as a control, were added to each well. Plates were incubated at 37°C for 16 h. Wells then washed twice with PBS buffer and the remaining biomass was determined by staining with crystal violet (0.1% w/v) for 15 minutes, followed by a gentle wash with tap water and de-staining in acetic acid (33% v/v). Absorbance was measured at a wavelength of 595 nm. All the assays were performed using three biological replicates.

Additionally, the ability of proteins to prevent biofilm formation was tested using a conventional broth microdilution technique. Two-fold dilutions of the proteins (0.02-50 µM) in TSBg were added to TC Microwell 96U w/lid nunclon DSI plates (Thermo Scientific, NUNC, Madrid, Spain). Each well was previously inoculated with 106 CFU/well of S. *aureus* 15981. Plates were incubated at 37°C for 24 hours. Biofilm formation was quantified by crystal violet staining as described above.

### Statistical analysis.

The SPSS Statistics for Windows V. 22.0 (IBM Corp.) package was used to determine differences of activity among the lytic proteins. One-way analysis of variance (ANOVA) followed by the Student-Newman-Keuls was used to determine differences among the activity of each protein at a level of significance p<0.05. In other hand, student t-test was performed to compare the differences between the treated and untreated bacterial culture at a level of significance p<0.05. The data were expressed as the mean ± standard deviation of three biological replicates.

### II. RESULTS

### S. aureus endolysins.

Initially, two endolysins encoded by two bacteriophages infecting S. *aureus* were identified. These endolysins (parental endolysins) will be used as source of the chimeric endolysins. Endolysin LysH5 is encoded by orf61 of phage philPLA88 genome. Endolysin LysRODI is encoded by orf57 of phage phiIPLA-RODI genome.

Bioinformatic analysis using BLASTP and HMMER allowed to determine that LysRODI and LysH5 show a modular organization consisting of two enzymatic domains (EADs) that catalyze peptidoglycan degradation (an N-terminal cysteine, histidine-dependent amino hydrolase/peptidase (CHAP), and a centrally located N-acetylmuramoyl-L-alanine amidase), and a cell wall binding domain (CBD) (an SH3b located at the C-terminal). These domains are separated by amino acid sequences that are called linkers. Linkers are also present at the beginning and at the end of the protein.

### Improving the expression and purification of two endolysins against S. aureus.

The genes codifying for the parental *endolysins* (LysH5 and LysRODI) were cloned into the pET21a expression vector and transformed into *E. coli* BL21 (DE3) for expression and further purification of the proteins. Nevertheless, after the purification process, the proteins were not soluble, the concentration of purified protein ranging from 0.3 to 0.6 mg/mL. To solve this issue, the genes codifying for the parental *endolysins* were optimized for expression in *E. coli,* resulting in a DNA sequence different to that deposited in the databases (supplementary material S1). The new synthetic genes were also cloned into pET21a and transformed into *E. coli* BL21 (DE3) for expression and further purification of the proteins. These new constructs resulted in completely soluble proteins after the purification process. The amount of the purified proteins ranged from 0.8 to 2 mg/mL.

### The chimeric lytic protein ClyRODI-H5 shows increased activity compared to the parental endolysins.

From the optimized nucleotide sequences that codify the parental *endolysins* (LysRODI and LysH5), a chimeric lytic protein (ClyRODI-H5) was obtained by domain shuffling. ClyRODI-H5 was designed using combinatory of the catalytic (CHAP domain) and the cell wall binding domains (SH3b) of the wild type *endolysins* LysRODI and LysH5, respectively. Prior to designing the *in silico* gene that codify for the chimeric protein, the predicted tridimensional structures of the parental *endolysins* were obtained using the software Phyre2. To ensure a proper folding of the chimeric lytic protein ClyRODI-H5, the CHAP domain of LysRODI was chosen including the amino acids from the beginning of the protein and 5 amino acids after the domain. The Phyre2 analyses revealed that there is a predicted alpha helix in the linker region preceding the SH3b domain of LysH5. Thus, the SH3b region chosen includes all the amino acids until the end of the protein and 59 amino acids before the domain that contains the complete alpha helix. Nucleotide and amino acid sequences of ClyRODI-H5 are included in the supplementary material S2. The chimeric lytic protein was successfully cloned into an expression vector derived from pNIC28-Bsa4 using the VersaTile technology (under patent by KU Leuven). ClyRODI-H5 was purified reaching an amount of 2 mg/mL of protein.

After purification, proteins LysRODI, LysH5 and ClyRODI-H5 were assessed for antimicrobial activity against *S*. *aureus* Sa9 by using the turbidity reduction assay. The specific lytic activity of each protein (ΔOD6OO × minutes⁻¹ × µM⁻¹) showed an activity of ClyRODI-H5 increased by 3 and 24-fold compared with the parental endolysins LysRODI and LysH5, respectively (Figure 1). This higher specific activity is indicative of a faster elimination of bacteria than that caused by the parental endolysins. In addition, the activity of the protein ClyRODI-H5 was compared with that of the lysostaphin (a bacteriocin with high activity against *S*. *aureus)* and the specific lytic activity was further calculated (ΔDO600 × minutes⁻¹ × µM⁻¹). ClyRODI-H5 showed an activity about 24-fold higher than lysostaphin (Figure 1).

### Influence of physicochemical conditions on the activity of chimeric protein.

To analyze the stability of parental and chimeric proteins, the influence of two environmental parameters (temperature and pH) on their specific lytic activity was determined. The effect of temperature was tested by incubation of proteins during 30 minutes in a range of 40°C-90°C. All the proteins were completely inactivated over 50ºC. It is remarkable that LysH5 retained its activity at 40ºC, while the activity of LysRODI decreased 7-fold at the same temperature. ClyRODI-H5 was also inactivated at 40ºC.

The activity of the proteins was tested at different pHs (3 to11). The parental endolysins (LysRODI and LysH5) retained their activity at a pH ranging from 5 to 9 and were completely inactivated at pH 3 and 11. Interestingly, the chimeric protein ClyRODI-H5 retained its activity in a broader range of pH (3-11). Moreover, a slightly increase of activity was observed (1.2- fold) for this protein at pH 5.

The influence of different ions (KCI, MgCl₂, NaCl, MnCl₂, ZnCl₂, CaCl₂) on the lytic activity was also evaluated. LysRODI and LysH5 were completely inactivated in the presence of MnCl₂ and ZnCI2. LysRODI was also inactivated by CaCl₂, while this ion increased 1.3-fold the activity of LysH5. MgCl₂ also increased the activity of LysH5 (1.3-fold). On the contrary, the activity of the chimeric protein ClyRODI-H5 was negatively influenced by the presence of all the ions, since a decrease ranging from 1.7 to 4-fold was observed. Of note, the protein was completely inactivated only in the presence of ZnCl₂.

### Spectrum of activity of parental and chimeric proteins.

Overlooking a future application as antimicrobials, proteins were tested against a collection of staphylococcal species, including *S*. *aureus* MRSA, from different origin. Minimum inhibitory concentration (MIC) was calculated for each strain and lysostaphin was used as a positive control (Table 1). LysH5 and ClyRODI-H5 were active only against S. *aureus* strains in the concentration range tested. The MIC values for ClyRODI-H5 were lower than those determined for LysH5, and similar to those obtained for LysRODI against S. *aureus.* Besides, LysRODI and lysostaphin are able to lyse all the tested staphylococcal species, the MIC values for lysostaphin being slightly lower.

### Chimeric protein ClyRODI-H5 lyses staphylococcal cells as fast as wild type LysRODI.

To determine the killing rate of ClyRODI-H5, time-kill curve experiments were performed using *S*. *aureus* Sa9 cultures (106 CFU/ml) treated with equimolar amount (0.1 µM) of each protein, and the number of viable bacteria was determined at different times (2 - 60 minutes). ClyRODI-H5 showed similar antibacterial activity than the parental endolysin LysRODI, with a reduction by 3.4 and 4.2 log units after 2 minutes of treatment (Figure 2). Of note, viable counts decreased up to 6 log units (under the detection limit) after 5 min of incubation in the presence of these two proteins. This represents a 2-fold increase of the lytic activity compared with lysostaphin and 5-fold increase compared with LysH5 (Figure 2). LysH5 did not achieve a complete elimination of the bacteria at the concentration and period of time tested.

### The parental endolysins and ClyRODI-H5 do not trigger staphylococcal resistance.

To assess the ability of *S*. *aureus* to develop resistance against the phage lytic proteins, we initially attempted to select mutants in liquid medium by serial subcultures of S. *aureus* Sa9 in the presence of sub-inhibitory concentrations of the proteins. Lysostaphin was used as a positive control. After 10 rounds of exposure, MIC values were determined and compared with the ones obtained previously (Table 2). As expected, the MIC values of lysostaphin increased more than 32-fold, whereas serial subcultures in liquid medium failed to select resistant variants for LysRODI, LysH5, or ClyRODI-H5.

| | **MIC (µM)** | | | |
|---|---|---|---|---|
| | **LysRODI** | **LysH5** | **ClyRODI-H5** | **Lysostaphin** |
| **Control** | 0.57 | 3.83 | 1.84 | 0.43 |
| **Treatment at sub-inhibitory concentrations** | 0.49±0.18 | 3.15±0.12 | 1.72±0.16 | >13.8* |

**Table 2.** Resistance development in liquid medium. Minimal Inhibitory Concentration (MIC) repeated exposure assay using 1:2 serial dilutions of each protein added to 5×10⁶ CFU/well of S. *aureus* Sa9. Cells surviving at ½ MIC were used as an inoculum for each subsequent round of exposure. Values represent mean ± standard deviation of three independent assays. Asterisk indicates statistical difference between the treated and control values (p<0.05), according to the Student t-test.

### Increased anti-biofilm properties of the chimeric protein.

Finally, the anti-biofilm properties of the proteins were assessed to both prevent biofilm formation and remove preformed biofilms on polystyrene surfaces. Proteins, including lysostaphin, were added to the wells using a two-fold microdilution technique (0.02-50 µM final concentration) prior to inoculation with *S*. *aureus* 15981 strain. Biofilms were grown at 37ºC for 24 hours. ClyRODI-H5 showed a similar activity than LysRODI and lysostaphin in preventing the biofilm formation (>98%). The minimum concentration to achieve this level of biofilm prevention was 0.7 µM (Figure 3). At the same concentration, LysH5 prevented the biofilm formation by 71%.

The chimeric protein ClyRODI-H5 was much active against preformed *S*. *aureus* 15981 biofilms than the parental proteins, and even than lysostaphin. Treatment of 8 hours-old biofilms with 2.5 µM of protein was able to remove 65% of the total biomass (Figure 4). This represents an increase of antibiofilm activity of 7-fold compared with lysostaphin.

## Claims

1. A chimeric protein comprising:
(a) a first amino acid sequence comprising the CHAP catalytic domain of the endolysin LysRODI from phage PhilPLA-RODI, and
(b) a second amino acid sequence comprising the SH3b cell wall binding domain of the endolysin LysH5 from phage philPLA88,
wherein the first amino acid sequence (a) is bound to the second amino acid sequence (b); and wherein the (i) stability and (ii) lytic activity of the chimeric protein against *Staphylococcus sp.* are improved when compared to those of the parental endolysins.

2. Chimeric protein according to claim 1, wherein the amino acid sequence of the CHAP catalytic domain comprises an amino acid sequence with a sequence identity of at least 80, 85, 90, 95, 96, 97, 98, 99 or 100% to SEQ ID NO: 1.

3. Chimeric protein according to claim 1 or 2, wherein the first amino acid sequence comprises the sequence SEQ ID NO: 2.

4. Chimeric protein according to any one of claims 1 to 3, wherein the amino acid sequence of the SH3b cell wall binding domain comprises an amino acid sequence with a sequence identity of at least 80, 85, 90, 95, 96, 97, 98, 99 or 100% to SEQ ID NO: 3.

5. Chimeric protein according to any one of claims 1 to 4, wherein the second amino acid sequence comprises the sequence SEQ ID NO: 4.

6. Chimeric protein according to any one of claim 1 to 5 comprising the amino acid sequence SEQ ID NO: 5.

7. An isolated polynucleotide comprising a nucleotide sequence encoding a chimeric protein according to any one of claims 1 to 6, preferably, the nucleotide sequence encoding a chimeric protein comprises the sequence SEQ ID NO: 6.

8. A vector comprising a polynucleotide according to claim 7.

9. A host cell comprising a chimeric protein according to any one of claims 1 to 6, a polynucleotide according to claim 7 and/or a vector according to claim 8, preferably, the host cell is *Escherichia coli.*

10. A composition comprising a chimeric protein according to any one of claims 1 to 6, a polynucleotide according to claim 7, a vector according to claim 8 and/or a host cell according to claim 9.

11. A chimeric protein according to any one of claims 1 to 6, a polynucleotide according to claim 7, a vector according to claim 8, a host cell according to claim 9 and/or a composition according to claim 10 for use as a medicament.

12. A chimeric protein according to any one of claims 1 to 6, a polynucleotide according to claim 7, a vector according to claim 8, a host cell according to claim 9 and/or a composition according to claim 10 for use in the treatment and/or prevention of an infection of *Staphylococcus* sp, preferably, *Staphylococcus aureus,* more preferably, S. *aureus* multi-drug resistant (MDR).

13. Use of a chimeric protein according to any one of claims 1 to 6, a polynucleotide according to claim 7, a vector according to claim 8, a host cell according to claim 9 and/or a composition according to claim 10 for removing, inhibiting and/or preventing *Staphylococcus* sp. biofilm formation on abiotic surfaces, or for removing and/or preventing the *Staphylococcus* sp. contamination of foods and/or abiotic surfaces, preferably, the *Staphylococcus* sp. is *Staphylococcus aureus,* more preferably, S. *aureus* multi-drug resistant (MDR).

14. A method for producing a chimeric protein according to any one of claims 1 to 6 comprising
(i) transforming a host cell with a polynucleotide according to claim 7, or a vector according to claim 8,
(ii) culturing the host cell resulting from step (a) under proper conditions for expressing the polynucleotide according to claim 7, or the vector according to claim 8, and optionally,
(iii) recovering the chimeric protein.

15. A kit comprising chimeric protein according to any one of claims 1 to 6, a polynucleotide according to claim 7, a vector according to claim 8, a host cell according to claim 9 and/or a composition according to claim 10.

## Patentansprüche

1. Chimäres Protein, umfassend:
(a) eine erste Aminosäuresequenz, umfassend die katalytische CHAP-Domäne des Endolysins LysRODI aus der Phage PhilPLA-RODI, und
(b) eine zweite Aminosäuresequenz, umfassend die SH3b-Zellwand-Bindungsdomäne des Endolysins LysH5 aus der Phage philPLA88,
wobei die erste Aminosäuresequenz (a) an die zweite Aminosäuresequenz (b) gebunden ist; und wobei die (i) Stabilität und (ii) lytische Aktivität des chimären Proteins gegen *Staphylococcus sp.* im Vergleich zu denen der elterlichen Endolysine verbessert sind.

2. Chimäres Protein nach Anspruch 1, wobei die Aminosäuresequenz der katalytischen Domäne von CHAP eine Aminosäuresequenz mit einer Sequenzidentität von mindestens 80, 85, 90, 95, 96, 97, 98, 99 oder 100 % der SEQ ID NR: 1 umfasst.

3. Chimäres Protein nach Anspruch 1 oder 2, wobei die erste Aminosäuresequenz die Sequenz SEQ ID NR: 2 umfasst.

4. Chimäres Protein nach einem der Ansprüche 1 bis 3, wobei die Aminosäuresequenz der wandbindenden Domäne von SH3b eine Aminosäuresequenz mit einer Sequenzidentität von mindestens 80, 85, 90, 95, 96, 97, 98, 99 oder 100 % der SEQ ID NR: 3 umfasst.

5. Chimäres Protein nach einem der Ansprüche 1 bis 4, wobei die zweite Aminosäuresequenz die Sequenz SEQ ID NR: 4 umfasst.

6. Chimäres Protein nach einem der Ansprüche 1 bis 5, umfassend die Aminosäuresequenz SEQ ID NR: 5.

7. Isoliertes Polynukleotid, umfassend eine Nukleotidsequenz, die ein chimäres Protein nach einem der Ansprüche 1 bis 6 codiert, wobei vorzugsweise die Nukleotidsequenz, die ein chimäres Protein codiert, die Sequenz SEQ ID NO: 6 umfasst.

8. Vektor, umfassend ein Polynukleotid nach Anspruch 7.

9. Wirtszelle, umfassend ein chimäres Protein nach einem der Ansprüche 1 bis 6, ein Polynukleotid nach Anspruch 7 und/oder eine Vektor nach Anspruch 8, wobei die Wirtszelle vorzugsweise *Escherichia coli* ist.

10. Zusammensetzung, umfassend ein chimäres Protein nach einem der Ansprüche 1 bis 6, ein Polynukleotid nach Anspruch 7, einen Vektor nach Anspruch 8 und/oder eine Wirtszelle nach Anspruch 9.

11. Chimäres Protein nach einem der Ansprüche 1 bis 6, ein Polynukleotid nach Anspruch 7, einen Vektor nach Anspruch 8, eine Wirtszelle nach Anspruch 9 und/oder eine Zusammensetzung nach Anspruch 10 zur Verwendung als ein Medikament.

12. Chimäres Protein nach einem der Ansprüche 1 bis 6, ein Polynukleotid nach Anspruch 7, einen Vektor nach Anspruch 8, eine Wirtszelle nach Anspruch 9 und/oder eine Zusammensetzung nach Anspruch 10 zur Verwendung bei der Behandlung und/oder Prävention einer Infektion mit *Staphylococcus* sp, vorzugsweise *Staphylococcus aureus,* mehr bevorzugt S. *aureus,* multiresistent (MDR).

13. Verwendung eines chimären Proteins nach einem der Ansprüche 1 bis 6, eines Polynukleotids nach Anspruch 7, eines Vektors nach Anspruch 8, einer Wirtszelle nach Anspruch 9 und/oder einer Zusammensetzung nach Anspruch 10 zum Entfernen, Hemmen und/oder Verhindern der *Staphylococcus sp.-*Biofilmbildung auf abiotischen Oberflächen oder zum Entfernen und/oder Vorbeugen des *Staphylococcus* sp.-Kontamination von Lebensmitteln und/oder abiotischen Oberflächen, wobei vorzugsweise *Staphylococcus sp. Staphylococcus aureus,* mehr bevorzugt S. *aureus,* multiresistent (MDR) ist.

14. Verfahren zum Herstellen eines chimären Proteins nach einem der Ansprüche 1 bis 6, umfassend
(i) Transformieren einer Wirtszelle mit einem Polynukleotid nach Anspruch 7 oder einem Vektor nach Anspruch 8,
(ii) Züchten der aus Schritt (a) resultierenden Wirtszelle unter geeigneten Bedingungen zum Exprimieren des Polynukleotids nach Anspruch 7 oder des Vektors nach Anspruch 8 und wahlweise,
(iii) Rückgewinnen des chimären Proteins.

15. Kit, umfassend das chimäre Protein nach einem der Ansprüche 1 bis 6, ein Polynukleotid nach Anspruch 7, einen Vektor nach Anspruch 8, eine Wirtszelle nach Anspruch 9 und/oder eine Zusammensetzung nach Anspruch 10.

## Revendications

1. Protéine chimérique comprenant :
(a) une première séquence d'acides aminés comprenant le domaine catalytique CHAP de l'endolysine LysRODI provenant du phage PhiIPLA-RODI et
(b) une seconde séquence d'acides aminés comprenant le domaine de liaison à la paroi cellulaire SH3b de l'endolysine LysH5 provenant du phage philPLA88,
dans laquelle la première séquence d'acides aminés (a) est liée à la seconde séquence d'acides aminés (b) ; et dans laquelle (i) la stabilité et (ii) l'activité lytique de la protéine chimérique vis-à-vis de *Staphylococcus sp.* sont améliorées par comparaison avec celles des endolysines parentes.

2. Protéine chimérique selon la revendication 1, dans laquelle la séquence d'acides aminés du domaine catalytique CHAP comprend une séquence d'acides aminés ayant une identité de séquence d'au moins 80, 85, 90, 95, 96, 97, 98, 99 ou 100 % avec SEQ ID N° : 1.

3. Protéine chimérique selon la revendication 1 ou 2, dans laquelle la première séquence d'acides aminés comprend la séquence SEQ ID N° : 2.

4. Protéine chimérique selon l'une quelconque des revendications 1 à 3, dans laquelle la séquence d'acides aminés du domaine de liaison à la paroi cellulaire SH3b comprend une séquence d'acides aminés ayant une identité de séquence d'au moins 80, 85, 90, 95, 96, 97, 98, 99 ou 100 % avec SEQ ID N° : 3.

5. Protéine chimérique selon l'une quelconque des revendications 1 à 4, dans laquelle la seconde séquence d'acides aminés comprend la séquence SEQ ID N°: 4.

6. Protéine chimérique selon l'une quelconque des revendications 1 à 5 comprenant la séquence d'acides aminés SEQ ID N° : 5.

7. Polynucléotide isolé comprenant une séquence nucléotidique codant pour une protéine chimérique selon l'une quelconque des revendications 1 à 6, de préférence, la séquence nucléotidique codant pour une protéine chimérique comprend la séquence SEQ ID N° : 6.

8. Vecteur comprenant un polynucléotide selon la revendication 7.

9. Cellule hôte comprenant une protéine chimérique selon l'une quelconque des revendications 1 à 6, un polynucléotide selon la revendication 7 et/ou un vecteur selon la revendication 8, de préférence, la cellule hôte est *Escherichia coli.*

10. Composition comprenant une protéine chimérique selon l'une quelconque des revendications 1 à 6, un polynucléotide selon la revendication 7, un vecteur selon la revendication 8 et/ou une cellule hôte selon la revendication 9.

11. Protéine chimérique selon l'une quelconque des revendications 1 à 6, polynucléotide selon la revendication 7, vecteur selon la revendication 8, cellule hôte selon la revendication 9 et/ou composition selon la revendication 10 destinés à être utilisés comme médicament.

12. Protéine chimérique selon l'une quelconque des revendications 1 à 6, polynucléotide selon la revendication 7, vecteur selon la revendication 8, cellule hôte selon la revendication 9 et/ou composition selon la revendication 10 destinés à être utilisés dans le traitement et/ou la prévention d'une infection à *Staphylococcus* sp, de préférence, *à Staphylococcus aureus,* plus préférablement, à S. *aureus* multirésistant aux médicaments (MDR).

13. Utilisation d'une protéine chimérique selon l'une quelconque des revendications 1 à 6, d'un polynucléotide selon la revendication 7, d'un vecteur selon la revendication 8, d'une cellule hôte selon la revendication 9 et/ou d'une composition selon la revendication 10 pour l'élimination, l'inhibition et/ou la prévention de la formation de biofilm de *Staphylococcus* sp. sur des surfaces abiotiques ou pour l'élimination et/ou la prévention de la contamination d'aliments et/ou de surfaces abiotiques par *Staphylococcus* sp., de préférence, le *Staphylococcus* sp. est *Staphylococcus aureus,* plus préférablement, S. *aureus* multirésistant aux médicaments (MDR).

14. Procédé de production d'une protéine chimérique selon l'une quelconque des revendications 1 à 6 comprenant
(i) la transformation d'une cellule hôte avec un polynucléotide selon la revendication 7, ou un vecteur selon la revendication 8,
(ii) la culture de la cellule hôte résultant de l'étape (a) dans des conditions appropriées pour exprimer le polynucléotide selon la revendication 7, ou le vecteur selon la revendication 8, et éventuellement,
(iii) la récupération de la protéine chimérique.

15. Kit comprenant une protéine chimérique selon l'une quelconque des revendications 1 à 6, un polynucléotide selon la revendication 7, un vecteur selon la revendication 8, une cellule hôte selon la revendication 9 et/ou une composition selon la revendication 10.
